# EUROPEAN PATENT SPECIFICATION

(11) **EP 4 463 445 B1**
(45) Date of publication and mention of the grant of the patent: **01.10.2025**
(21) Application number: 22840334.1
(22) Date of filing: 23.12.2022
(51) Int. Cl.: C07D 303/38, C07D 405/12, A61P 25/00, A61K 31/336

(54) **COMPOUNDS AND USE THEREOF FOR THE TREATMENT OF DISEASES ASSOCIATED WITH IMPAIRED ?-GALACTOSIDASE ACTIVITY**
VERBINDUNGEN UND IHRE VERWENDUNG ZUR BEHANDLUNG VON ERKRANKUNGEN IM ZUSAMMENHANG MIT GESTÖRTER ALPHA-GALACTOSIDASE-AKTIVITÄT
COMPOSÉS ET LEUR UTILISATION POUR LE TRAITEMENT DE MALADIES ASSOCIÉES À UNE ACTIVITÉ DE ?-GALACTOSIDASE ALTÉRÉE

(30) Priority: 13.01.2022 EP 22151412
(43) Date of publication of application: 20.11.2024
(73) Proprietor: Dorphan S.A., 1066 Epalinges (CH)
(72) Inventor: ROCHAT, Virginie, 1700 Fribourg (CH); MARTI, Roger, 1700 Fribourg (CH); DESCLOUX, Mélanie, 1066 Epalinges (CH); DEMOTZ, Stéphane, 1066 Epalinges (CH); MUTEL, Vincent, 1066 Epalinges (CH)
(74) Representative: KATZAROV S.A.
(86) International application number: PCT/IB2022/062740
(87) International publication number: WO 2023/135480

(56) References cited:
- WO-A1-2019/211205
- GREGORY HOOK ET AL: "Cathepsin B is a New Drug Target for Traumatic Brain Injury Therapeutics: Evidence for E64d as a Promising Lead Drug Candidate", FRONTIERS IN NEUROLOGY, vol. 6, 1 January 2015 (2015-01-01), pages 1 - 27, XP055706009, DOI: 10.3389/fneur.2015.00178
- SHI MING W. ET AL: "Electrostatic complementarity in pseudoreceptor modeling based on drug molecule crystal structures: the case of loxistatin acid (E64c)", NEW JOURNAL OF CHEMISTRY, vol. 39, no. 3, 1 January 2015 (2015-01-01), GB, pages 1628 - 1633, XP093021037, ISSN: 1144-0546, DOI: 10.1039/C4NJ01503G

## Description

### Technical Field

The present invention relates to new cathepsin B inhibitors, which are effective in therapy, and in particular in the treatment of diseases associated with an impaired activity of the β-galactosidase, such as GM-1 gangliosidosis, Morquio syndrome type B, Chediak-Higashi Syndrome, Galactosialidosis, Metachromatic leukodystrophy, Gaucher Disease, Alzheimer disease and traumatic brain injury.

### Background Art

The human β-galactosidase (EC 3.2.1.23, also called lactase) is a lysosomal enzyme classified as a member of the glycoside hydrolase family and is present in both animals and plants as well as in many microorganisms. Its function is to catalyse the hydrolysis of the terminal β-D-galactose residue from various substrates, including lactose, oligosaccharides, glycolipids and glycoproteins, by hydrolysis. This enzyme is especially known for its ability to hydrolyse lactose into glucose and galactose.

Among diseases associated with impaired β-galactosidase activity, one can cite two lysosomal storage diseases: the GM1-gangliosidosis and the mucopolysaccharidosis (MPS) type IVB (also known as Morquio syndrome type B). More specifically, β-gal is known to degrade the glycosphingolipid GM1-ganglioside and the muccopolysaccharide keratan sulfate. Pathogenic mutations on the gene encoding this enzyme may lead to:
(i) a strong deformation of the catalytic site resulting in an inactive enzyme;
(ii) slighter impairment of the structure within the catalytic site or its neighbourhood resulting in a diminished enzyme activity; and
(iii) a structural change that prevents a proper folding of the polypeptide, resulting in a premature degradation of the enzyme.

Any of those lead to a lower amount of functional β-gal in cells and therefore in an accumulation of substrate in the organism. Storage of GM1 leads to GM1-gangliosidosis while accumulation of keratan sulfate relates to Morquio syndrome type B.

In the particular case of the GM1-gangliosidosis, the reduced activity of β-gal impairs GM1 breakdown. This results in a substrate accumulation within the lysosome, which is experiencing a disruption in its functionality. Since the highest concentration of GM1 is located in the CNS, excessive accumulation of GM1 will lead to the death of neuronal cells, resulting in a neurodegenerative course. The devastating effects of GM1-gangliosidosis on the central nervous system make it an essential need to find therapeutic approaches that are able to cross the blood-brain barrier. Because β-gal is used to hydrolyse the β-galactosyl residue, its deficiency leads to the storage of other substrates, like glycolipids GA1, oligosaccharides from glycoproteins and glycosaminoglycans. Depending on the organ where these substrates are produced, the β-gal defect will also result in some non-neurological manifestations, such as dysmorphic face, disorders in bone development (known as dysostosis) or an enlargement of both the spleen and the liver (a condition called hepatosplenomegaly which shows an enlarged abdomen).

Several therapeutic strategies have been implemented to treat GM1-gangliosidosis. However, conventional therapies fail to achieve treatment of the symptoms in the central nervous system and therefore provide no benefit in terms of reduction of neurodegeneration. Hematopoietic stem cells therapy (HSCT) was unsuccessfully attempted in a patient with infantile form (N. Brunetti-Pierri and F. Scaglia, GM1 gangliosidosis: Review of clinical, molecular, and therapeutic aspects, Mol. Genet. Metab., 2008, 94(4), 391-396). Recombinant enzymes have also been used, but are not able to cross the blood-brain barrier. Substrate reduction therapy (SRT), which consists in administration of small molecules that partially inhibits the biosynthesis of the accumulated substrates, was attempted on three patients with types II and III GM1 gangliosidosis. In particular, the administration of Miglustat made it possible to slow-down the degenerative course, or even to reverse it (F. Deodato et al., The treatment of juvenile/adult GM1- gangliosidosis with Miglustat may reverse disease progression, Metab. Brain Dis., 2017, 32(5), 1529-1536). Gene therapy was conclusive in both mice (R. C. Baek et al., AAV-Mediated Gene Delivery in Adult GM1-Gangliosidosis Mice Corrects Lysosomal Storage in CNS and Improves Survival, PLoS One, 2010, 5(10), e13468) and feline (V. J. McCurdy et al., Sustained normalization of neurological disease after intracranial gene therapy in a feline model, Sci. Transl. Med., 2014, 6(231), 231-279) models through intracerebroventricular injection of an engineered adeno-associated virus/GLB1 vector. Many chaperoned were developed until now, with Migalastat being the first one to be tested. Since it was not specific enough for the GM1-gangliosidosis, other iminosugars were developed, noticeably N-octyl-4-epi-β-valienamine (NOEV) and 5N,6S-(N'-butyliminomethylidene)-6-thio-1-deoxygalactonojirimycin (6S-NBI-DGJ) that showed promising results in the murine model (Y. Suzuki et al., Therapeutic chaperone effect of N-Octyl 4- Epi-β-valienamine on murine GM1-gangliosidosis, Mol. Genet. Metab., 2012, 106(1), 92-98 and T. Takai et al., A Bicyclic 1-Deoxygalactonojirimycin Derivative as a Novel Pharmacological Chaperone for GM1 Gangliosidosis, Mol. Ther., 2013, 21(3), 526-532). Unfortunately, chaperone therapy is based on the residual activity of the misfolded β-gal, resulting in a total inefficacy of such treatment in cases where the enzyme is totally inactive.

Therefore, there is need to further develop therapeutic strategies targeting GM1-gangliosidosis, which would allow to reach the central nervous system, and which would be applicable even to cases wherein the β-gal is totally inactive.

The Morquio syndrome type B (also known as mucopolysaccharidosis type IVB, MPS IVB) is caused by some specific mutations in the GLB1 gene encoding for β-gal, which catalyses the degradation of some mucopolysaccharides. This defect results into an accumulation of keratan sulphate that mainly leads to skeletal dysplasia and frequent respiratory infections. Patients exhibit a normal development of their neurocognitive functions, but in some advanced stages of the disease, a spinal cord compression might occur due to the skeletal deformities leading to an impairment of the central nervous system. Beyond bone problems, MPS IVB is characterized by some ocular manifestation, such as corneal clouding, retinopathy or glaucoma, and cardiorespiratory disease.

Not only is there currently no approved treatment for MPS IVB, but also the lack of knowledge of the disease and its clinical manifestations, linked to the extreme rarity of this condition, limits the possibility of a well-designed clinical trial, even if some molecules showed promising results as pharmaceutical chaperones. As for GM1-gangliosidosis, the only available therapy so far is symptomatic. Therefore, there is an acute need for developing curative therapeutic strategies.

Other diseases have been shown to be associated with impaired activity of the β-galactosidase, such as Chediak-Higashi Syndrome, Galactosialidosis, Metachromatic leukodystrophy, Gaucher Disease, Alzheimer disease and traumatic brain injury.

One of the key systems impacted by a number of such diseases is the central nervous system. This might come from the fact that the brain, due to its limited regeneration capacity and neuron sensitivity, is highly vulnerable, namely towards LSDs, and in particular GM1-gangliosidosis. Damages to the central nervous system are also significant in the case of Alzheimer disease and traumatic brain injury. This sensitivity of the central nervous system causes a major problem in terms of treatment because the entry into the brain is highly protected by the blood-brain barrier, which only allows for small molecules to pass through, and makes it impossible, for example, to deliver enzymes in the brain via the bloodstream. The treatment of diseases causing neurological symptoms is therefore a significant challenge.

Among publications related to activity of β-galactosidase, it was described that cathepsin B (catB) had a regulatory role in the maturation and degradation of β-galactosidase (Y. Okamura-Oho et al., Maturation and degradation of β-galactosidase in the post-Golgi compartment are regulated by cathepsin B and a non-cysteine protease, FEBS Lett., 1997, 419(2-3), 231-234). These authors identified that inhibition of cathepsin B promoted the activity of β-galactosidase. In view of developing new therapies for diseases involving a reduced activity of the β-galactosidase or the absence of activity of the β-galactosidase, it would be desired to identify new inhibitors of cathepsin B. It would further be advantageous to provide such inhibitors that exhibit properties making them suitable for use as a drug, namely inhibitors exhibiting good chemical stability, permeability through the cell membrane, solubility, metabolic stability, plasma stability and/or transporters affinity.

One of the first identified catB inhibitor was the oxirane E64, a natural epoxysuccinyl dipeptide found in *Aspergillus japonicus* that irreversibly and non-selectively inhibits cathepsins (K. Hanada et al., Isolation and Characterization of E-64, a New Thiol Protease Inhibitor, Agric. Biol. Chem., 1978, 42(3), 523-528). The chemical structure of E64 is provided in [Table 1].

Since the discovery of E64, a number of analogues have been developed. The most famous representatives of this class of compounds are provided in [Table 1] below and have been disclosed in the following publications: T. Otsuka et al., WF14865A and B, new cathepsins B and L inhibitors produced by Aphanoascus fulvescens. I. Taxonomy, production, purification and biological properties, J. Antibiot. (Tokyo)., 2000, 53(5), 449-458, A. J. Barrett et al., L-trans-Epoxysuccinyl-leucylamido(4- guanidino)butane (E-64) and its analogues as inhibitors of cysteine proteinases including cathepsins B, H and L, Biochem. J., 1982, 201(1), 189-198, M. Murata et al., Novel epoxysuccinyl peptides. Selective inhibitors of cathepsin B, in vitro, FEBS Lett., 1991, 280(2), 307-310, D. J. Buttle and J. Saklatvala, Lysosomal cysteine endopeptidases mediate interleukin 1-stimulated cartilage proteoglycan degradation, Biochem. J., 1992, 287(2), 657-661, D. J. Buttle et al., CA074 methyl ester: a proinhibitor for intracellular cathepsin B, Arch. Biochem. Biophys., 1992, 299(2), 377-380, and N. Schaschke et al., Substrate/propeptide-derived endo- epoxysuccinyl peptides as highly potent and selective cathepsin B inhibitors, FEBS Lett., 1998, 421(1), 80-82.

**[Table 1]**

| Inhibitor | Structure |
|---|---|
| E64c (also named simply E64) | 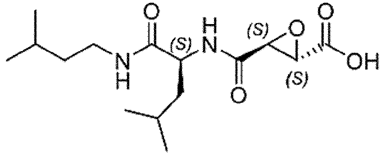 |
| E64d | 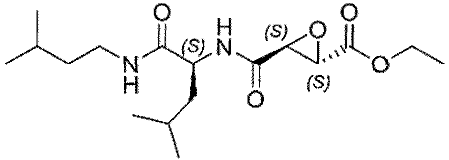 |
| CA030 | 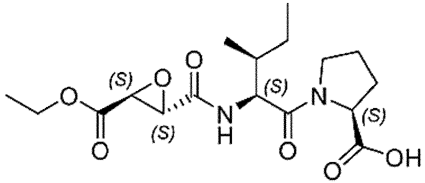 |
| CA074 | 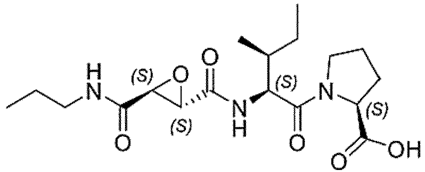 |
| CA074Me | 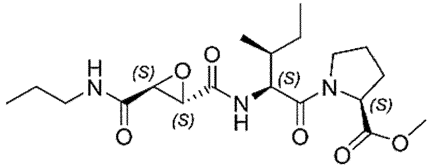 |
| NS134 | 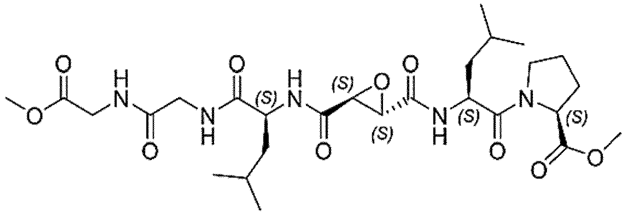 |

Currently, the only molecule that has demonstrated catB inhibition properties, while exhibiting good absorption, distribution, metabolism, and excretion is E64d, known as Loxistatin. This compound, an ester prodrug of E64c, has been the subject of clinical studies in Japan as a treatment for muscular dystrophies. Despite discontinuing the study due to unclear effectiveness, these 3-year trials (including pediatric patients) yielded extensive pharmacokinetics (PK) and pharmacodynamics (PD) data, while also demonstrating the non-toxicity for human (E. Satoyoshi, Therapeutic Trials on Progressive Muscular Dystrophy, Intern. Med., 1992, 31(7), 841-846, G. Hook et al., Cathepsin B is a New Drug Target for Traumatic Brain Injury Therapeutics: Evidence for E64d as a Promising Lead Drug Candidate, Front. Neurol., 2015, 6, 178 and T. Miyahara et al., Phase I study of EST, a new thiol protease inhibitor. The 1st report: Safety and pharmacokinetics at single administration, Rinsho yakuri/Japanese J. Clin. Pharmacol. Ther., 1985, 16(2), 357-365).

Unfortunately, this compound presents a low penetration of the blood-brain barrier, which significantly reduces the chances of reaching a therapeutic target in the central nervous system (CNS). This makes this compound of low relevance for pathologies associated with the presence of catB in the brain, like GM-1 gangliosidosis, Alzheimer disease and traumatic brain injury.

There is therefore a need to develop new compounds capable of irreversibly inhibiting catB. There is a further need to identify such compounds, which are further capable of reaching the central nervous system by penetrating the blood-brain barrier.

### Summary of Invention

In a first aspect, the invention provides a compound of Formula I or Formula II 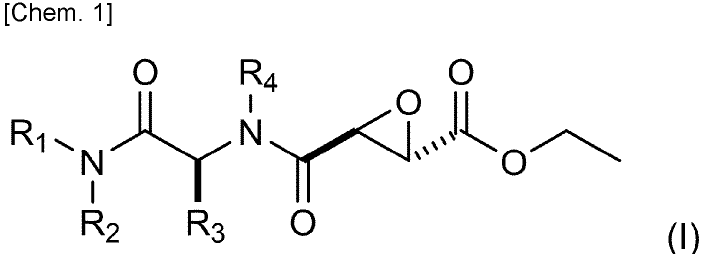 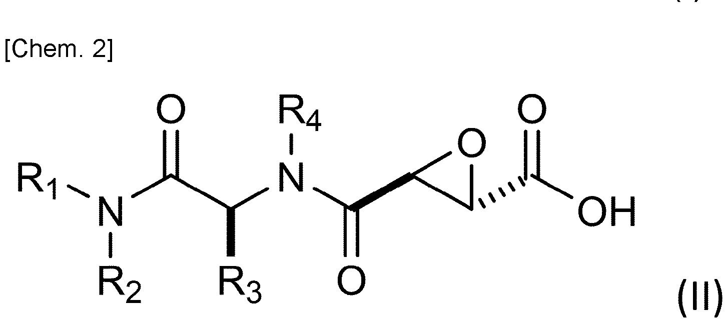 wherein
R₁ is selected from 3-methylbutyl, 2-methoxyethyl, 2-fluorophenylmethyl, 3-(piperidin-1-yl)propyl and 2-(pyridin-2-yl)ethyl.
R₂ is selected from hydrogen and methyl;
R₃ is selected from 2-methylpropyl and 2,2,2-trifluoroethyl; and
R₄ is hydrogen;
said compound being characterized in that, when R₁ is 3-methylbutyl,
   i. R₃ is not 2-methylpropyl; or
   ii. R₃ is 2-methylpropyl and R₂ is methyl,
said compound being further characterized in that, when R₃ is 2,2,2-trifluoroethyl, R₂ is hydrogen.

In a second aspect, the present invention provides a compound of the invention, for use in therapy, preferably for the treatment of diseases associated with impaired β-galactosidase activity.

In a third aspect, the present invention provides a compound of a compound of Formula I or Formula II 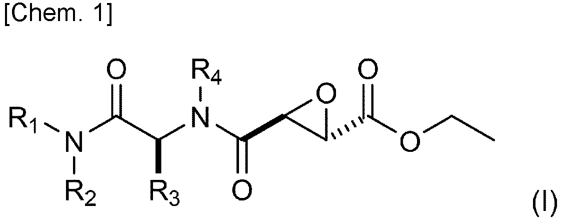 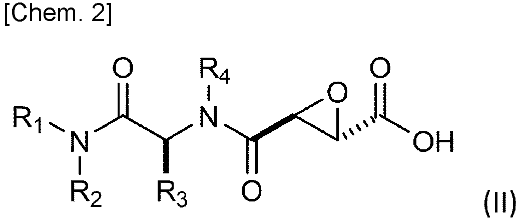 wherein
R₁ is selected from 3-methylbutyl, 2-methoxyethyl, 3-methoxypropyl, 2-fluorophenylmethyl, 3-(piperidin-1-yl)propyl and 2-(pyridin-2-yl)ethyl.
R₂ is selected from hydrogen and methyl;
R₃ is selected from 2-methylpropyl and 2,2,2-trifluoroethyl; and
R₄ is hydrogen;
said compound being characterized in that, when R₁ is 3-methylbutyl,
   i. R₃ is not 2-methylpropyl; or
   ii. R₃ is 2-methylpropyl and R₂ is methyl,
said compound being further characterized in that, when R₃ is 2,2,2-trifluoroethyl, R₂ is hydrogen
for use in a method for treating a disease associated with impaired β-galactosidase activity.

In a fourth aspect, the present invention provides a process for producing a compound according to the invention, comprising the steps of:
a) deprotonating the compound of Formula III with a base, such as N,N-Diisopropylethylamine (DIPEA); 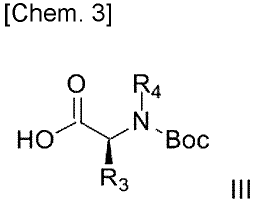
b) reacting the deprotonated compound obtained in step a) with 1-[Bis(dimethylamino)methylene]-1H-1,2,3- triazolo[4,5-b]pyridinium 3-oxid hexafluoro-phosphate (HATU) in basic media;
c) reacting the compounds obtained in step b) with tetramethylurea (TMU) in basic media;
d) reacting the compound obtained in step c) with an amine of Formula IV in basic media, such as to form the dipeptide of Formula V; 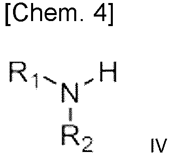 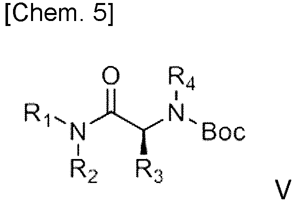
e) removing the Boc group from the compound of Formula V by reacting such compound of Formula V with trifluoroacetic acid in acidic media to obtain the compound of Formula VI; 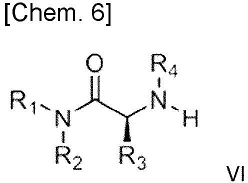
f) deprotonating the compound of Formula VI with a base, such as DIPEA;
g) reacting the deprotonated compound obtained in step f) with HATU in basic media;
h) reacting the compounds obtained in step g) with TMU in basic media;
i) reacting the compounds obtained in step h) with a compound of Formula VII, such as to obtain a compound of Formula I; and 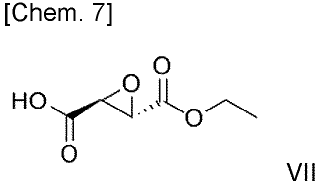
j) optionally hydrolysing the ester moiety of the compound of Formula I, such as to obtain a compound of Formula II.

### Brief Description of Drawings

[Fig.1] is a graph representing inhibition of cathepsin B (catB) activity in pork liver homogenate for VRO006-hydrol, VRO052-hydrol, VRO059_hydrol, VRO042_hydrol, VRO047_hydrol, VRO073_hydrol, VRO109_hydrol and VRO244_hydrol (compounds with suitable inhibitory activity (IC50 ≤100 nM).
[Fig. 2] is a graph representing inhibition of cathepsin B (catB) activity in pork liver homogenate for VRO001_hydrol, VRO035_hydrol, VRO082_hydrol, VRO119_hydrol, VRO243_hydrol, VRO073_hydrol, VRO109_hydrol and VRO244_hydrol (compounds with unsuitable inhibitory activity (IC50 > 100 nM)).
[Fig. 3] is a graph representing inhibition of human cathepsin B by E64c, VRO052-hydrol and VRO059-hydrol in a whole cell assay.
[Fig. 4] represents the mechanism of the formation of the dipeptide in steps a) to d) of the process of the present invention.

### Detailed Description

The present inventors have advantageously developed new compounds that are active as inhibitors of cathepsin B and therefore beneficial in the treatment of diseases associated with impaired β-galactosidase (β-gal) activity. Furthermore, the identified compounds have properties that make them particularly suitable for use as a medicament. In particular, such compounds are characterized by one or more of the following advantageous properties:
a) good absorption, which makes it possible for the compounds to reach the systemic bloodstream after administration to the patient;
b) good distribution to the different tissues/organs to be treated;
c) appropriate metabolism, wherein the compound is metabolized by the body in a way that maintains the activity of the compound and does not generate harmful metabolites; and/or
d) appropriate excretion of the compound and its metabolites.

The compounds of the invention are further advantageous in that they are suitable to cross the blood-brain barrier, which makes it possible to treat diseases impacting the central nervous system. This represents a critical improvement, over the prior art compound E64d. Furthermore, the compounds of the invention exhibit good selectivity towards inhibition of catB over other enzymes of the cathepsin family.

The compounds of the present invention are of Formula I or Formula II 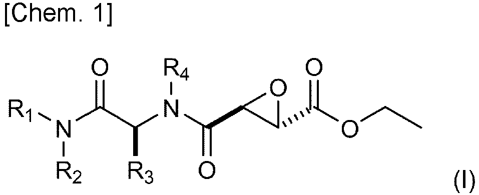 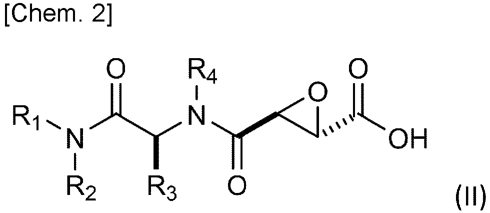 wherein
R₁ is selected from 3-methylbutyl, 2-methoxyethyl, 2-fluorophenylmethyl, 3-(piperidin-1-yl)propyl and 2-(pyridin-2-yl)ethyl.
R₂ is selected from hydrogen and methyl;
R₃ is selected from 2-methylpropyl and 2,2,2-trifluoroethyl; and
R₄ is hydrogen;
said compound being characterized in that, when R₁ is 3-methylbutyl,
   i. R₃ is not 2-methylpropyl; or
   ii. R₃ is 2-methylpropyl and R₂ is methyl,
said compound being further characterized in that, when R₃ is 2,2,2-trifluoroethyl, R₂ is hydrogen.

The structure of the compounds of the invention consists of two blocks, with a dipeptide structure including both nitrogen atoms and the radicals R₁, R₂ and R₄, said dipeptide structure being linked to an epoxy warhead. As in the case of the prior art E64d, the compounds of Formula I of the present invention are prodrugs, which are hydrolyzed in the gastrointestinal tract, upon enteral administration. The ester moiety in the far end of the epoxy warhead is cleaved to release the free acid. It is in this cleaved form that the compounds are therapeutically active. Therefore, the compounds of Formula I are advantageously for use as prodrugs, while compounds of Formula II are advantageously for use as therapeutic agents.

Examples of compounds according to the invention are provided in [Table 2] below:

**[Table 2]**

| Compound reference | Structure | Chemical name |
|---|---|---|
| VRO047 | 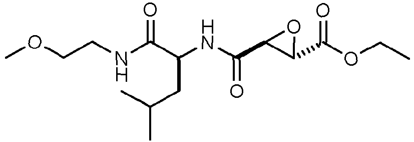 | Ethyl (2S,3S)-3-(((S)-1-((2-2-methoxyethyl)amino)-4-methyl-1-oxopentan-2-yl)carbamoyl)oxirane-2-carboxylate |
| VRO006 | 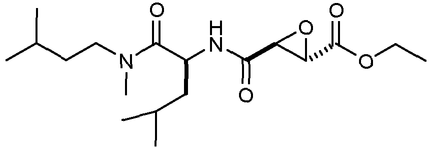 | Ethyl (2S,3S)-3-(((S)-1-(isopentyl(methyl)amino)-4-methyl-1-oxopentan-2-yl)carbamoyl)oxirane-2-carboxylate |
| VRO073 | 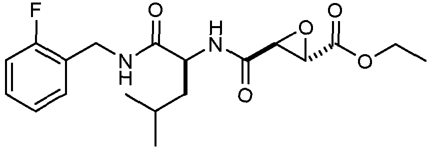 | Ethyl (2S,3S)-3-(((S)-1-((2-fluorobenzyl)amino)-4-methyl-1-oxopentan-2-yl)carbamoyl)oxirane-2-carboxylate |
| VRO059 | 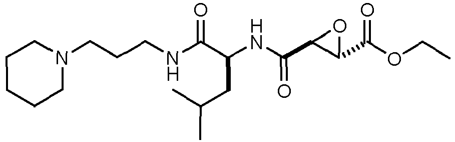 | Ethyl (2S,3S)-3-(((S)-4-methyl-1-oxo-1-((3-(piperidin-1-yl)propyl)amino)pentan-2-yl)carbamoyl)oxirane-2-carboxylate |
| VRO052 | 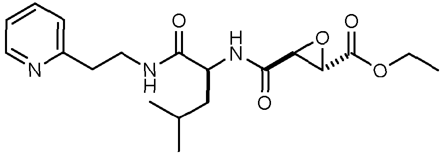 | Ethyl (2S,3S)-3-(((S)-4-methyl-1-oxo-1-((2-(pyridin-2-yl)ethyl)amino)pentan-2-yl)carbamoyl)oxirane-2-carboxylate |
| | | |
| VRO109 | 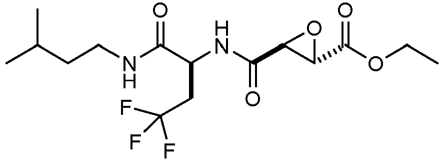 | Ethyl (2S,3S)-3-(((S)-4,4,4-trifluoro-1-(isopentylamino)-1-oxobutan-2-yl)carbamoyl)oxirane-2-carboxylate |
| VRO244 | 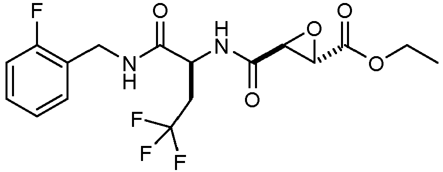 | Ethyl (2S,3S)-3-(((S)-4,4,4-trifluoro-1-((2-fluorobenzyl)amino)-1-oxobutan-2-yl)carbamoyl)oxirane-2-carboxylate |

Examples of compounds of Formula II according to the present invention are the counterparts of the compounds provided in [Table 2], in the form of the free acid. Such compounds include (2S,3S)-3-(((S)-1-((2-2-methoxyethyl)amino)-4-methyl-1-oxopentan-2-yl)carbamoyl)oxirane-2-carboxylic acid (VRO047_hydrol), (2S,3S)-3-(((S)-1-(isopentyl(methyl)amino)-4-methyl-1-oxopentan-2-yl)carbamoyl)oxirane-2-carboxylic acid (VRO006_hydrol), (2S,3S)-3-(((S)-1-((2-fluorobenzyl)amino)-4-methyl-1-oxopentan-2-yl)carbamoyl)oxirane-2-carboxylic acid (VRO073_hydrol), (2S,3S)-3-(((S)-4-methyl-1-oxo-1-((3-(piperidin-1-yl)propyl)amino)pentan-2-yl)carbamoyl)oxirane-2-carboxylic acid (VRO059_hydrol), (2S,3S)-3-(((S)-4-methyl-1-oxo-1-((2-(pyridin-2-yl)ethyl)amino)pentan-2-yl)carbamoyl)oxirane-2-carboxylic acid (VRO052_hydrol), (2S,3S)-3-(((S)-4,4,4-trifluoro-1-(isopentylamino)-1-oxobutan-2-yl)carbamoyl)oxirane-2-carboxylic acid (VRO109_hydrol) and (2S,3S)-3-(((S)-4,4,4-trifluoro-1-((2-fluorobenzyl)amino)-1-oxobutan-2-yl)carbamoyl)oxirane-2-carboxylic acid (VRO244_hydrol).

The compound of the invention are advantageously for use in therapy.

The compounds of the invention are also advantageously for use in the treatment of a disease associated with impaired β-galactosidase activity. In addition to the compounds according to the present invention recited above, a compound of Formula I or Formula II, wherein R₁ is 3-methoxypropyl and wherein R₂, R₃ and R₄ are as described above, is also for use in the treatment of a disease associated with impaired β-galactosidase activity. Preferably such compound of Formula I for use in the treatment of a disease associated with impaired β-galactosidase activity is ethyl (2S,3S)-3-(((S)-1-((3-3-methoxypropyl)amino)-4-methyl-1-oxopentan-2-yl)carbamoyl)oxirane-2-carboxylate (VRO042) having the structure provided below and such compound of Formula II for use in the treatment of a disease associated with impaired β-galactosidase activity is the free acid counterpart (2S,3S)-3-(((S)-1-((3-3-methoxypropyl)amino)-4-methyl-1-oxopentan-2-yl)carbamoyl)oxirane-2-carboxylic acid (VRO042_hdrol).

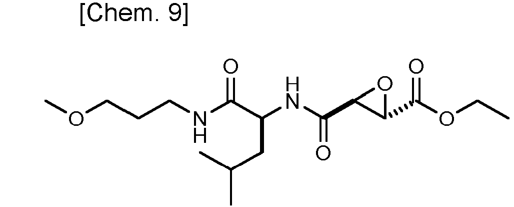

Diverse properties of the compounds of Formula II make them more suitable to cross the blood-brain barrier than the prior art catB inhibitors such as E64b and its known derivatives. In particular, the present compounds are advantageously characterized by
a) a higher lipophilicity, for example expressed as the cLogP, leading to a better passive permeation of the blood-brain barrier.
b) a reduced ability to form hydrogen-bonds with other compounds, as a result of the reduction of the number of hydrogen-bond donor and/or hydrogen-bond acceptor atoms and/or as a result of the formation of intramolecular hydrogen bonds, thus leading to an improved permeation and a reduced affinity for plasma glycoproteins;
c) a more peptidomimetic structure, leading to an improved metabolic stability and/or an enhanced affinity towards catB with a better occupancy of the S2 pocket of catB.

As demonstrated by the present inventors, the specific radicals R₁, R₂ and R₃ described above are advantageous in that they improve the drug-likeness and the ability to cross the blood-brain barrier of the compounds of Formula II compared to E64c, while active as inhibitors of catB. It has also been shown that it is essential that R₄ be a hydrogen atom. Without wishing to be being bound by theory, it is believed that this hydrogen atom is involved in a hydrogen-bond with the protein.

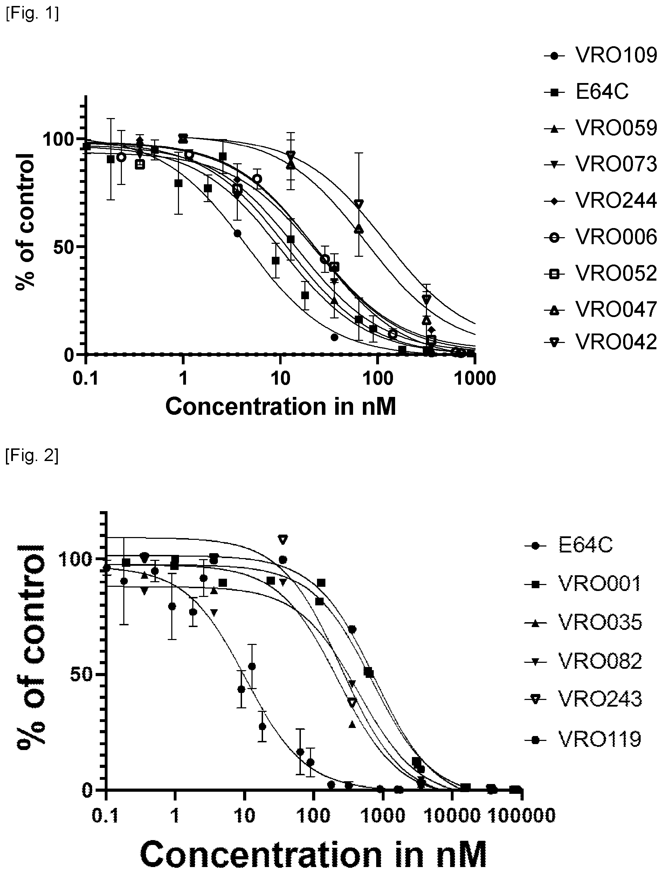

Unlike the hydrogen in position R₄, the hydrogen in position R₂ can be replaced by a methyl without harming the inhibitor activity of the compound, as demonstrated on [Fig. 1], wherein it can be seen that the ability of VR006_hydrol, having a methyl in position R₂, to inhibit isolated catB is similar to that of E64c. This modification of the prior art structure is further advantageous in that the replacement of the hydrogen atom by a methyl makes VRO006_hydrol more lipophilic than E64c (cLogP around 2), removes one hydrogen donor in the molecule, and reduces the topological polar surface area of the molecule (TPSA) to under 90 Å². Due to these differences, VRO006_hydrol exhibits more drug-like properties, is more suitable to enter the cell through the cell membrane and is more suitable than E64c to cross the blood-brain barrier.

The present inventors have also identified that it was possible to advantageously use more bulky radicals in position R₁ such as those recited herein, without harming the interaction between the compound and the S3 pocket of catB (see [Fig. 1]). This results in improved drug-likeness and in an improved ability to cross the blood-brain barrier over the prior art E64c, while maintaining efficient inhibition of catB. This was the case with R₁ being 2-methoxyethyl (VRO047_hydrol) and with R₁ being 3-methoxypropyl (VRO042_hydrol). The same was observed with R₁ being 2-(pyridin-2-yl)ethyl (VRO052_hydrol) or 3-(piperidin-1-yl)propyl (VRO059_hydrol), as these compounds both exhibit an inhibitory activity against isolated catB almost similar to E64c. Even the bulky radical 2-(2-fluorophenyl)ethyl in position R₁ (VRO073_hydrol) allowed to maintain the inhibitory activity against isolated catB, this compound having only slightly reduced catB inhibitory activity compared to E64c. Such minimal reduction of the inhibition of the enzyme is compensated by the benefit in terms of drug-likeness, and in terms of ability to reach the interior of the cell through the cell membrane and cross the blood-brain barrier.

However, not all bulky substituents are suitable for maintaining sufficient inhibition of catB and, for example, the present inventors demonstrated that the bulky and highly electronegative 3,3,3-trifluoroethyl significantly reduced the efficiency of VRO035_hydrol in the enzymatic assay against isolated catB (see [Fig. 2]).

Particularly preferred radicals in position R₁ are 2-(pyridin-2-yl)ethyl and 3-(piperidin-1-yl)propyl. Indeed, compounds VRO052_hydrol and VRO059_hydrol, in which these radicals were present in position R₁, exhibited significantly improved inhibitory activity against catB in a whole cell assay. Without wishing to be bound by theory, it is believed that this is due to the increased lipophilicity of these compounds, which more easily cross the cell membrane. The in-vivo efficiency of compounds bearing these radicals in position R₁ is thus expected to be higher than that of E64c.

With respect to R₃, which interacts with the S2 pocket of catB, the present inventors also achieved the replacement of 3-methylbutyl present in E64d by diverse radicals improving the drug-likeness of the compound. Most bulky radicals negatively impacted the inhibitory activity of the compound, such as for example the pyridinyl moiety (VRO119_hydrol), as demonstrated by [Fig. 2]. However, replacement of 3-methylbutyl in position R₃ by 2,2,2-trifluoroethyl unexpectedly caused a significant increase in the inhibitory activity of VRO109_hydrol against catB, as shown in [Fig. 1]. This trifluoroethyl moiety is further advantageous in that it does not contain any oxygen or nitrogen atom, which have the undesired property of being able to generate hydrogen bonds with other molecules, and in that it significantly increases the lipophilicity and the drug-likeness of VRO109_hydrol compared to E64c.

The combination of a suitable radical in position R₁ with a suitable radical in position R₃ leads to compounds that still exhibit suitable inhibitory activity, as shown on [Fig. 2], wherein compound VRO244_hydrol exhibits inhibitory activity only slightly lower than the prior art E64c, but is characterized by an increased lipophilicity and ability to enter the cell and cross the blood-brain barrier, which makes VRO244_hydrol more suitable as a drug than E64c.

It is however key not to combine the bulky 2,2,2-trifluoroethyl in position R3 with a methyl in position R2, as this specific combination deprives VRO243_hydrol from inhibitory activity on the isolated enzyme (see [Fig. 2]).

The advantages of the compounds of Formula II identified above make the corresponding compounds of Formula I advantageous prodrugs.

Due to their ability to inhibit catB, the compounds of Formula I and of Formula II, as described above, can advantageously be used in the treatment of a disease associated with impaired β-galactosidase activity. This is due to previously demonstrated regulatory role of catB in the maturation and degradation of β-galactosidate, as explained in detail in the background section.

Examples of diseases associated with impaired β-galactosidase activity include GM-1 gangliosidosis, Morquio syndrome type B, Chediak-Higashi Syndrome, Galactosialidosis, Metachromatic leukodystrophy, Gaucher Disease, Alzheimer disease and traumatic brain injury. Preferably, the compounds of the invention are for use in the treatment of a disease selected from GM-1 gangliosidosis, Morquio syndrome type B, Chediak-Higashi Syndrome, Galactosialidosis, Metachromatic leukodystrophy and Gaucher Disease, more preferably GM-1 gangliosidosis and Morquio syndrome type B and most preferably GM-1 gangliosidosis.

In other words, the invention relates to the use of a compound of the invention for the manufacture of a medicament. Preferably it relates to the use of a compound of Formula I or of Formula II, as described above, for the manufacture of a medicament for the treatment of a disease associated with impaired β-galactosidase activity, preferably a medicament for the treatment of a disease selected from GM-1 gangliosidosis, Morquio syndrome type B, Chediak-Higashi Syndrome, Galactosialidosis, Metachromatic leukodystrophy, Gaucher Disease, Alzheimer disease and traumatic brain injury, more preferably a disease selected from GM-1 gangliosidosis, Morquio syndrome type B, Chediak-Higashi Syndrome, Galactosialidosis, Metachromatic leukodystrophy and Gaucher Disease, even more preferably a disease selected from GM-1 gangliosidosis and Morquio syndrome type B and most preferably GM-1 gangliosidosis.

In still other words, the invention relates to a method of treatment of a disease in a patient in need thereof, comprising administering a compound of the invention to such patient. It also relates to a method of treatment of a disease associated with impaired β-galactosidase activity in a patient in need thereof comprising administering a compound of Formula I or of Formula II, as described above, to such patient. More preferably such disease is selected from GM-1 gangliosidosis, Morquio syndrome type B, Chediak-Higashi Syndrome, Galactosialidosis, Metachromatic leukodystrophy, Gaucher Disease, Alzheimer disease and traumatic brain injury, more preferably such disease is selected from GM-1 gangliosidosis, Morquio syndrome type B, Chediak-Higashi Syndrome, Galactosialidosis, Metachromatic leukodystrophy and Gaucher Disease, even more preferably such disease is selected from GM-1 gangliosidosis and Morquio syndrome type B and most preferably GM-1 gangliosidosis.

In a particular aspect, the compounds of Formula I or of Formula II are for use in a method of inhibiting catB. In other words, the invention relates to the use of a compound of Formula I or of Formula II for inhibiting catB. In still other words, it relates to a method of inhibiting catB in a subject, comprising administration of such compounds to the subject.

In another particular aspect, the compound of Formula I or of Formula II is administered to a patient in need thereof in combination with at least one additional compound that is active to treat one of the diseases associated with impaired β-galactosidase mentioned above. In a preferred aspect, such additional compound is selected from a pharmacological chaperone of the β-galactosidase, a pharmacological chaperone of the glucocerebrosidase, a calcium channel blocker and/or a glucosylceramide synthase inhibitor, and mixtures thereof. In another preferred aspect, such additional compound is selected from the group consisting of N-substituted 5-amino-1-hydroxymethyl-cyclopentanetriols, N-octyl-4-epi-beta-valienamine (NOEV), ambroxol, *cis*-(+)-[2-(2-dimethylaminoethyl)-5-(4-methoxyphenyl)-3-oxo-6-thia-2-azabicyclo[5.4.0]undeca-7,9,11-trien-4-yl]ethanoate (Diltiazem), [(3S)-1-azabicyclo[2.2.2]octan-3-yl] *N*-[2-[2-(4-fluorophenyl)-1,3-thiazol-4-yl]propan-2-yl]carbamate (Venglustat), an iminosugar and mixtures thereof. More preferably, such iminosugar is preferably selected from the group consisting of N-butyl-deoxygalactonojirimycin (Migalastat), 4-epi-isofagomine, 5a-C-pentyl 4-epi-isofagomine, 5a-*C*-methyl 4-epi-isofagomine, 1,5-dideoxy-1,5-imino-(L)-ribitol (DIR), 5-C-alkyl-imino-L-ribitol, N-(dansylamino)hexylaminocarbonylpentyl-1,5-dideoxy-1,5-imino-D-galactitol, 5N,6S-(N'-butyliminomethylidene)-6-thio-1-deoxygalactonojirimycin (6S-NBI-DGJ), N-nonyl-deoxygalactonojirimycin, N-butyldeoxynojirimycin (Miglustat), isofagomine (Afegostat), AZ-3102 and mixtures thereof.

The compounds of Formula I of the present invention are preferably administered by enteral administration. By enteral administration, it is intended any means of administration the compound of Formula I is conveyed into the gastrointestinal tract. Enteral administration of the compounds of Formula I is advantageous, as such compounds are hydrolysed to the free acid in the gastrointestinal tract. The compounds of Formula II of the present invention can be administered by any route and are preferably administered by an administration route that is not enteral administration.

In a preferred aspect, the compound of Formula I or of Formula II is administered in the form of a pharmaceutical composition, preferably comprising a pharmaceutically acceptable carrier, diluent and/or excipient.

The term "carrier" refers to an organic or inorganic component, of a natural or synthetic nature, in which the active component is combined in order to facilitate, enhance or enable application. According to the invention, the term "carrier" also includes one or more compatible solid or liquid fillers, diluents or encapsulating substances, which are suitable for administration to a patient.

Possible carrier substances for parenteral administration are e.g. sterile water, Ringer, Ringer lactate, sterile sodium chloride solution, polyalkylene glycols, hydrogenated naphthalenes and, in particular, biocompatible lactide polymers, lactide/glycolide copolymers or polyoxyethylene/polyoxy- propylene copolymers.

The term "excipient" when used herein is intended to indicate all substances which may be present in a composition described herein and which are not active ingredients such as, e.g., carriers, binders, lubricants, thickeners, surface active agents, preservatives, emulsifiers, buffers, flavoring agents, or colorants.

The excipient of the composition can be any pharmaceutically acceptable excipient, including specific carriers able to target specific cells or tissues. As stated earlier, possible pharmaceutical compositions include those suitable for oral, rectal, topical, transdermal, buccal, sublingual, or parenteral (including subcutaneous, intramuscular, intravenous and intradermal) administration. For these formulations, conventional excipients can be used according to techniques well known by those skilled in the art. The compositions for parenteral administration are generally physiologically compatible sterile solutions or suspensions, which can optionally be prepared immediately before use from solid or lyophilized form. For oral administration, the composition can be formulated into conventional oral dosage forms such as tablets, capsules, powders, granules and liquid preparations, such as syrups, elixirs, and concentrated drops. Nontoxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents, which impart cohesive qualities to powdered materials, are also necessary. For example, starch, gelatine, sugars such as lactose or dextrose, and natural or synthetic gums can be used as binders. Disintegrants are also necessary in the tablets to facilitate break-up of the tablets. Disintegrants include starches, clays, celluloses, algins, gums and cross-linked polymers. Moreover, lubricants and glidants are also included in the tablets to prevent adhesion of the tablet material to surfaces in the manufacturing process and to improve the flow characteristics of the powder material during manufacture. Colloidal silicon dioxide is most commonly used as a glidant and compounds, such as talc or stearic acid are most commonly used as lubricants. For transdermal administration, the composition can be formulated into ointment, cream or gel form and appropriate penetrants or detergents could be used to facilitate permeation, such as dimethyl sulfoxide, dimethyl acetamide and dimethylformamide. For transmucosal administration, nasal sprays, rectal or vaginal suppositories can be used. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate.

The pharmaceutical composition may be formulated to substantially release the active drug immediately upon administration or at any predetermined time or a time period after administration.

The compound of Formula I or of Formula II is administered in an effective amount. The term "effective amount" refers to an amount necessary to obtain a physiological effect. The physiological effect may be achieved by one application dose or by repeated applications. The dosage administered may, of course, vary depending upon known factors, such as the physiological characteristics of the particular composition; the age, health and weight of the subject; the nature and extent of the symptoms; the kind of concurrent treatment; the frequency of treatment; and the effect desired and can be adjusted by a person skilled in the art. In a particular embodiment, the pharmaceutical composition comprises the compound of Formula I or of Formula II in an amount of 0.1 mg to 5 g, preferably in an amount of 1 mg to 2 g, more preferably in an amount of 10 mg to 1 g. In another particular embodiment, the compound of Formula I or of Formula II is administered in an amount of 0.1 mg to 5 g, preferably in an amount of 1 mg to 2 g, more preferably in an amount of 10 mg to 1 g per day.

The compounds of the invention can be produced by a process comprising forming an amid bond between two amino acids to produce a dipeptide structure and coupling the formed dipeptide with the epoxy warhead.

Therefore, the invention relates to a process for producing the compounds of the invention comprising:
a) deprotonating the compound of Formula III with a base, such as N,N-Diisopropylethylamine (DIPEA); 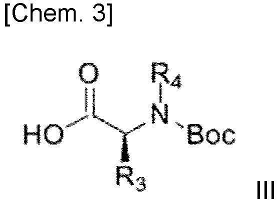
b) reacting the deprotonated compound obtained in step a) with 1-[Bis(dimethylamino)methylene]-1H-1,2,3- triazolo[4,5-b]pyridinium 3-oxid hexafluoro-phosphate (HATU) in basic media;
c) reacting the compounds obtained in step b) with tetramethylurea (TMU) in basic media;
d) Reacting the compound obtained in step c) with an amine of Formula IV in basic media, such as to form the dipeptide of Formula V; 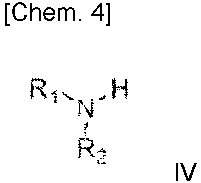 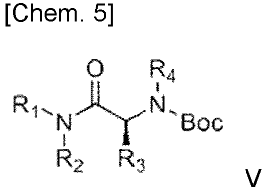
e) Removing the Boc group from the compound of Formula V by reacting such compound of Formula V with trifluoroacetic acid in acidic media to obtain the compound of Formula VI; 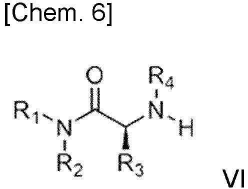
f) deprotonating the compound of Formula VI with a base, such as DIPEA;
g) reacting the deprotonated compound obtained in step f) with HATU in basic media;
h) reacting the compounds obtained in step g) with TMU in basic media; and
i) reacting the compounds obtained in step h) with a compound of Formula VII

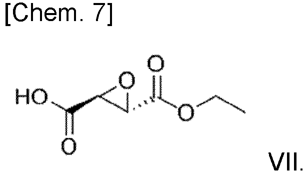

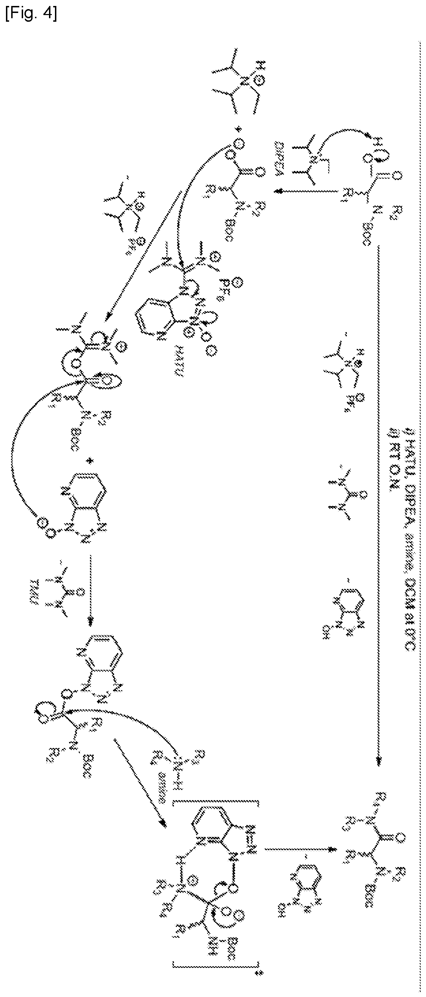

Steps a) to d) are sub-steps of the first stage of the synthesis of the compounds of the invention, wherein the dipeptide part of the structure is formed. This involves the formation of an amide bond between an acid and a base and is commonly called peptide coupling. Many coupling reagents can be used and are well-known to the person skilled in the art. However, HATU is particularly suitable as it is relatively tolerant towards different types of coupling starting materials. Preferably, such steps are performed in basic media and more preferably at 0°C. The mechanism to amide bond formation mediated by HATU is depicted in [Fig. 4]. It starts by the deprotonation of the acid by the base, which is preferably DIPEA. The formed carboxylate then attacks the electron-deficient carbon from HATU, thus forming an unstable O-acyl(tetramethyl)isouronium salt and releasing the 7-azabenzotriazole (OAt). The latter will immediately attack the isouronium salt to form tetramethylurea (TMU) and the OAt-activated ester. It is this active species that will undergo a nucleophile addition by the amine of Formula III. The definitive amide bond is then formed to produce the compound of Formula IV. Without wishing to be bound by theory, the mechanism probably goes through the formation of a 7-membered cyclic transition state that might stabilise the amine while the amide bond is formed.

In step e), the Boc protecting group is removed, in an acidic media, preferably with 25% TFA in DCM. The mechanism starts by the protonation of the t-butyl carbamate. This leads to the loss of the t-butyl cation and the formation of a carbamic acid. The carboxylate from TFA will then deprotonate the carbamic acid, thus releasing the free amine and forming one equivalent of carbon dioxide. During the work-up, a first acidic extraction allows to remove some organic impurities while the amine stays in the aqueous layer as a TFA salt. The free amine can be recovered in the organic layer upon basification.

In steps f) to i), the epoxy warhead is bound to the deprotected dipeptide structure. This is performed in a similar way as the formation of the dipeptide structure, since it also involves an amide bond formation. A compound of Formula I is obtained. Again, a variety of coupling agents can be used, which are well-known to the person skilled in the art. Such coupling agents include *N,N*'-Dicyclohexylcarbodiimide (DCC) or HATU. However, as DCC has been described as being allergenic, it is preferred to use HATU. HATU exhibited the same capacities and even gave better yields than DCC.

In cases wherein it is desired to form a compound of Formula II, the process comprises one further step of hydrolysing the ester moiety of the compound of Formula I, to release the free acid of Formula II. Conditions appropriate for such hydrolysis are well-known to the person skilled in the art. For example the compound of Formula I can be provided in a basic medium.

### Examples

### Analytical methods

¹H and ¹³C NMR as well as 2D-NMR (COSY, HSQC and HMBC) were obtained on a Bruker Ascend 300 MHz spectrometer. All measurements were performed at RT. Chemical shifts (δ, expressed in ppm) were referenced against solvent peak. all syntheses were followed by UPLC-MS using an ACQUITY UPLC system from Waters, equipped with a UV detector and coupled to an SQD2. The column was a UPLC BEH C18 (50 x 2.1 mm, 1.7 µm), the detection was performed at 214 nm, the analyses were run at 40°C with a flow rate of 0.5 mL/min and elution used 0.06% formic acid in water as eluent A and 0.06% formic acid in acetonitrile as eluant B. The gradient was going from 5 to 100% of B in 5 minutes, staying for 2 minutes at 100% of B, then re-equilibrated to 5% for 30 seconds.

The TLC analysis were all performed on Merck Silica Gel F254 plates and revealed under both 254 and 366 nm and then using hanessian's stain.

### Example 1: Synthesis of VRO006

Firstly, the dipeptide was formed. In a 25 mL round-bottomed flask, equipped with a magnetic stirrer, 500 mg of Boc-L-Leu (2.162 mmol, 1.0 eq.) and 10 mL of DCM were introduced, the resulting colourless solution was stirred at RT for 5 min. then cooled down to 0-5°C using an ice-bath. 1.051 g of HATU (3.243 mmol, 1.5 eq.) were added at 0-5°C and the reaction mixture was stirred for 5 min at this temperature. Then 312 mg of N,3-dimethylbutan-1-amine hydrochloride (2.270 mmol, 1.05 eq.) were introduced at 0- 5°C and the reaction mixture was stirred for 5 min at this temperature. Finally, 1.1 mL of DIPEA (6.485 mmol, 3.0 eq.) were added dropwise at 0-5°C. The turbid and slightly yellow mixture was stirred for 5 min at 0-5°C then the ice-bath was removed and the mixture was stirred O.N. at RT.

The reaction mixture, a clear yellowish solution was transferred into a separating funnel using 10 mL of DCM and washed with 50 mL of NaCl 2.6%, then AP was extracted once with 10 mL of DCM. OPs were combined and washed with 50 mL of NaCl 2.6%, then AP was extracted once with 10 mL of DCM. OPs were combined, washed once with 50 mL of HCl 0.25 M, once with 50 mL of NaHCO3 10% and finally with 50 mL of NaCl 11.6%. OP was dried over Na2SO4, filtered over a P3 sintered glass filter and concentrated to dryness into the rotavapor (40°C, 850-70 mbar). The material was dried under high vacuum (RT, 10-3 mbar) for 2h to yield 766 mg (raw yield: 112.7%) of an orange pasty material as 362-VRO006- 01-001 crude1#1.

The crude material was purified by CC using 40 g of silica gel (column with ø = 3.2 cm and h = 13.5 cm) and elution followed using TLC using heptane-EtOAc (7:3) as eluant; Rf = 0.44. The product was eluted with a gradient from pure heptane to heptane-EtOAc (6:4), fractions containing the desired product were combined and concentrated to dryness into the rotavapor (40°C, 200-50 mbar). The material was dried under high vacuum (RT, 10-3 mbar) for 1h to yield 610 mg (raw yield: 89.7%) of an almost colourless oil as 362-VRO006-01-001 CC1#1.

¹H -NMR (300 MHz, CDCl3) δ = 5.37 - 5.11 (m, 1H, 4), 4.73 - 4.54 (m, 1H, 5), 3.50 - 3.23 (m, 2H, 11), 3.03 (s, 2H, 10), 2.92 (s, 1H, 10), 1.55 (br. m, 4H, 6, 7, 13), 1.42 (s, 9H, 1, 2, 3), 1.40 - 1.30 (m, 2H, 12), 0.98 (dd, J=12.7, 6.4, 6H, 8, 9, 14, 15), 0.92 (dd, J=6.6, 2.5, 6H, 8, 9, 14, 15).

¹³C NMR (75 MHz, CDCl3) δ = 48.72 (5), 48.26 (5), 46.50 (11), 43.33 (12), 42.70 (12), 37.28 (6), 35.73 (6), 34.83 (10), 33.61 (10), 28.29 (1, 2, 3), 26.12 (7, 13), 25.92 (7, 13), 24.56 (7, 13), 23.46 (8, 9, 14, 15), 23.41 (8, 9, 14, 15), 22.51 (8, 9, 14, 15), 22.47 (8, 9, 14, 15), 22.43 (8, 9, 14, 15), 22.40 (8, 9, 14, 15), 21.75 (8, 9, 14, 15), 21.71 (8, 9, 14, 15).

The Boc protecting group was then removed as follows. In a 25 mL round-bottomed flask, equipped with a magnetic stirrer, 601 mg of 362-VRO006-01-001 CC1#1 (1.911 mmol, 1.0 eq.) and 2.1 mL of a solution of 10% HCl in EtOAc were introduced, the resulting colourless solution was stirred at RT for 3h30. The reaction mixture was transferred into a separating funnel using 25 mL of EtOAc and washed with 25 mL of water. AP was basified using 6 mL of NaOH 25% to reach pH > 10, then extracted 3 times with 25 mL of EtOAc. OPs were combined and washed using 25 mL of NaCl 11.6%. OP was dried over Na2SO4, filtered over a P3 sintered glass filter and concentrated to dryness into the rotavapor (40°C, 250-70 mbar). The material was dried under high vacuum (RT, 10-3 mbar) for 1h to yield 371 mg (raw yield: 90.6%) of an almost colourless oil as 362-VRO006-02-001 crude1#1. This material was used "as it" without further purification in the next step.

¹H-NMR (300 MHz, CDCl3) δ = 3.88 - 3.57 (m, 1H, 2), 3.33 (br. m, 2H, 8), 2.99 (s, 1H, 7), 2.93 (s, 1H, 7), 2.01 (br. s, J=18.9, 2H, 1), 1.95 - 1.77 (m, 1H, 3'), 1.42 (br. m, 5H, 3", 4, 9, 10), 1.09 - 0.79 (m, 12H, 5, 6, 11, 12).

Finally, the dipeptide was coupled to the epoxy warhead. In a 25 mL round-bottomed flask, equipped with a magnetic stirrer, 282 mg of 362-BB01-03-001 crude1#1 (1.759 mmol, eq.) and 5 mL of DCM were introduced, the resulting white suspension was stirred at RT for 5 min. then cooled down to 0-5°C using an ice-bath. 814 mg of HATU (2.512 mmol, 1.5 eq.) were added at 0-5°C and the reaction mixture was stirred for 5 min at this temperature. Then 359 mg of 362-VRO006-02-001 crude1#1 (1.675 mmol, 1.0 eq.) in solution in 5 mL of DCM were introduced at 0-5°C and the reaction mixture was stirred for 5 min at this temperature. Finally, 875 µL of DIPEA (5.024 mmol, 3.0 eq.) were added dropwise at 0-5°C. The resulting yellow mixture was stirred for 5 min at 0- 5°C then the ice-bath was removed and the mixture was stirred O.N. at RT.

The reaction mixture, a clear yellow solution, was transferred into a separating funnel using 10 mL of DCM and washed with 50 mL of NaCl 2.6%, then AP was extracted once with 10 mL of DCM. OPs were combined, and washed once with 50 mL NaCl 2.6%, then AP was extracted once with 10 mL of DCM. OPs were combined and washed once with 50 mL of HCl 0.25 M, once with 50 mL of NaHCO₃ 10% and once with 50 mL of NaCl 11.6%. OP was dried over Na₂SO₄, filtered over a P3 sintered glass filter and concentrated to dryness into the rotavapor (40°C, 220-30 mbar). The material was dried under high vacuum (RT, 10-3 mbar) for 2h to yield 701 mg (raw yield: 117.4%) of a pale yellowish oil as 362-VRO006-03- 001 crude1#1.

The crude material was purified by CC using 40 g of silica gel (column with ø = 3.2 cm and h = 13.5 cm) and elution followed using TLC using heptane-EtOAc (1:1) as eluant; R_{f} = 0.53. The product was eluted with a gradient from heptane-EtOAc (9:1) to heptane-EtOAc (1:1), fractions containing the desired product were combined and concentrated to dryness into the rotavapor (40°C, 200-50 mbar). The material was dried under high vacuum (RT, 10-3 mbar) for 2h30 to yield 334 mg (raw yield: 55.9%) of a colourless oil as 362-VRO006-03-001 CC1#1.

¹H-NMR (300 MHz, CDCl₃) δ = 6.84 (dd, J=16.2, 8.8, 1H, 5), 5.02 - 4.87 (m, 1H, 6), 4.37 - 4.17 (m, 2H, 2), 3.65 (dd, J=1.8, 0.8, 1H, 4), 3.46 (dd, J=1.8, 0.9, 1H, 3), 3.34 (br. m, 2H, 12), 3.04 (s, 2H, 11), 2.92 (s, 1H, 11), 1.48 (br. m, 6H, 7, 8, 13, 14), 1.31 (t, J=7.1, 3H, 1), 1.05 - 0.86 (m, 12H, 9, 10, 15, 16).

¹³C-NMR (75 MHz, CDCl₃) δ = 62.20 (2), 53.88 (4), 52.81 (3), 48.28 (12), 47.06 (6), 46.72 (6), 46.64 (12), 42.81 (7), 42.32 (7), 37.27 (13), 35.67 (13), 34.86 (11), 33.70 (11), 26.07 (8, 14), 25.94 (8, 14), 24.72 (9, 10, 15, 16), 23.44 (9, 10, 15, 16), 23.41 (9, 10, 15, 16), 22.50 (9, 10, 15, 16), 22.45 (9, 10, 15, 16), 22.38 (9, 10, 15, 16), 22.36 (9, 10, 15, 16), 21.69 (9, 10, 15, 16), 21.60 (9, 10, 15, 16), 14.00 (1).

### Example 2: Synthesis of VRO047

Firstly, the dipeptide was formed. In a 50 mL round-bottomed flask, equipped with a magnetic stirrer, 3.232 g of Boc-L-Leu (13.975 mmol, 1.05 eq.) and 35 mL of DCM were introduced, the resulting turbid solution was stirred at RT for 5 min. then cooled down to 0-5°C using an ice-bath. 6.471 g of HATU (19.946 mmol, 1.5 eq.) were added at 0-5°C and the reaction mixture was stirred for 5 minutes a this temperature. Then 1157 µL of 2-2-methoxyethylamine (13.309 mmol, 1.0 eq.) were introduced at 0-5°C and the reaction mixture was stirred for 5 min at this temperature. Finally, 4.6 mL of DIPEA (26.618 mmol, 2.0 eq.) were added dropwise at 0-5°C. The resulting turbid and yellowish solution was stirred for 5 min at 0-5°C then the ice-bath was removed and the mixture was stirred O.N. at RT.

The reaction mixture, a clear yellow solution, was transferred into a separating funnel using 10 mL of DCM and washed with 50 mL of NaCl 2.6%, then AP was extracted once with 10 mL of DCM. OPs were combined and washed once with 50 mL of HCl 0.5 M, once with 50 mL of NaHCO3 10% and finally with 50 mL of NaCl 11.6%. OP was dried over Na2SO4, filtered over a P3 sintered glass filter and concentrated to dryness into the rotavapor (40°C, 850-70 mbar). The material was dried under high vacuum (RT, 10-3 mbar) for 2h40 to yield 6.986 g (raw yield: 182.0%) of a pale yellow oil as 362- VRO047-01-001 crude1#1.

The crude material was purified by CC using 35 g of silica gel (column with ø = 3.2 cm and h = 11.8 cm) and elution followed using TLC using heptane-EtOAc (1:1) as eluant; Rf = 0.46. The product was eluted with a gradient from pure heptane to heptane-EtOAc (1:1), fractions containing the desired product were combined and concentrated to dryness into the rotavapor (40°C, 220-50 mbar). The material was dried under high vacuum (RT, 10-3 mbar) for 1h to yield 1.267 g (raw yield: 33.0) of a white sticky foam as 362-VRO047-01-001 CC1#1.

¹H-NMR (300 MHz, CDCl3) δ = 6.37 (br. s, 1H, 10), 4.88 (br. s, 1H, 4), 4.24 - 3.89 (m, 1H, 5), 3.50 - 3.38 (m, 4H, 11, 12), 3.35 (s, 3H, 13), 1.76 - 1.56 (m, 2H, 6", 7), 1.56 - 1.35 (m, 1H, 6'), 1.44 (s, 9H, 1, 2, 3), 0.93 (dd, J=6.2, 1.1, 6H, 8, 9).

Then the Boc protecting group was removed as follows. In a 25 mL round-bottomed flask, equipped with a magnetic stirrer, 1.267 g of 362-VRO047-01-001 CC1#1 (4.393 mmol, eq.) and 4.4 mL of a solution of 10% HCl in EtOAc were introduced, the resulting colourless solution was stirred at RT for 3h. The reaction mixture was transferred into a separating funnel using 25 mL of EtOAc and washed with 25 mL of water. AP was basified using 4.5 mL of NaOH 25% to reach pH > 10, then extracted 3 times with 25 mL of EtOAc. OPs were combined and washed using 25 mL of NaCl 11.6%. OP was dried over Na₂SO₄, filtered over a P3 sintered glass filter and concentrated to dryness into the rotavapor (40°C, 250-70 mbar). The material was dried under high vacuum (RT, 10-3 mbar) for 3h to yield 337 mg (raw yield: 40.7%) of a pale yellow oil as 362-VRO047-02-001 crude1#1. This material was used "as it" without further purification in the next step.

¹H-NMR (300 MHz, CDCl₃) δ = 7.49 (br. s, 1H, 7), 3.57 - 3.37 (m, 5H, 2, 8, 9), 3.36 (s, 3H, 10), 1.83 - 1.57 (m, 4H, 1, 3", 4), 1.47 - 1.28 (m, 1H, 3'), 0.95 (dd, J=8.4, 6.2, 6H, 5, 6).

Finally, the dipeptide was coupled to the epoxy warhead. In a 25 mL round-bottomed flask, equipped with a magnetic stirrer, 290 mg of 362-BB01-03-001 crude1#1 (1.813 mmol, 1.05 eq.) and 5 mL of DCM were introduced, the resulting white suspension was stirred at RT for 5 min. then cooled down to 0-5°C using an ice-bath. 839 mg of HATU (2.589 mmol, 1.5 eq.) were added at 0-5°C and the reaction mixture was stirred for 5 min at this temperature. Then 325 mg of 362-VRO047-02-001 crude1#1 (1.726 mmol, 1.0 eq.) in solution in 5 mL of DCM were introduced at 0-5°C and the reaction mixture was stirred for 5 min at this temperature. Finally, 601 µL of DIPEA (3.452 mmol, 2.0 eq.) were added dropwise at 0-5°C. The resulting mixture was stirred for 5 min at 0-5°C then the ice-bath was removed and the mixture was stirred O.N. at RT.

The reaction mixture, a clear yellow solution, was transferred into a separating funnel using 10 mL of DCM and washed with 50 mL of NaCl 5.8%, then AP was extracted once with 10 mL of DCM. OPs were combined, and washed once with 50 mL NaCl 5.8%, then AP was extracted once with 10 mL of DCM. OPs were combined and washed once with 50 mL of HCl 0.25 M, once with 50 mL of NaHCO₃ 10% and once with 50 mL of NaCl 11.6%. OP was dried over Na₂SO₄, filtered over a P3 sintered glass filter and concentrated to dryness into the rotavapor (40°C, 220-30 mbar). The material was dried under high vacuum (RT, 10-3 mbar) for 2h to yield 560 mg (raw yield: 98.2%) of a yellowish viscous solid as 362-VRO047- 03-001 crude1#1.

The crude material was purified by CC using 45 g of silica gel (column with ø = 3.2 cm and h = 15.2 cm) and elution followed using TLC using heptane-EtOAc (3:7) as eluant; R_{f} = 0.41. The product was eluted with a gradient from heptane-EtOAc (7:3) to heptane-EtOAc (2:8), fractions containing the desired product were combined and concentrated to dryness into the rotavapor (40°C, 200-40 mbar). The material was dried under high vacuum (RT, 10-3 mbar) for 2h to yield 220 mg (raw yield: 38.6%) of a slightly pale yellow solid as 362-VRO047-03-001 CC1#1.

¹H-NMR (300 MHz, CDCl₃) δ = 6.58 (d, J=8.6, 1H, 5), 6.23 (br. s, 1H, 11), 4.41 (td, J=8.5, 5.6, 1H, 6), 4.26 (qq, J=7.1, 3.6, 2H, 2), 3.69 (d, J=1.8, 1H, 4), 3.51 - 3.39 (m, 5H, 3, 12, 13), 3.36 (s, 3H, 14), 1.56 (br. m, J=23.2, 13.4, 9.5, 5.2, 3H, 7, 8), 1.31 (t, J=7.1, 3H, 1), 0.93 (dd, J=6.1, 2.9, 6H, 9, 10).

¹³C-NMR (75 MHz, CDCl₃) δ = 70.75 (13), 62.30 (2), 58.76 (14), 53.77 (4), 52.88 (3), 51.16 (6), 41.51 (7), 39.26 (12), 24.76 (8), 22.78 (9, 10), 22.05 (9, 10), 13.99 (1).

### Example 3: Synthesis of VRO052

Firstly, the dipeptide was formed. In a 50 mL round-bottomed flask, equipped with a magnetic stirrer, 2.235 g of Boc-L-Leu (9.661 mmol, 1.1 eq.) and 25 mL of DCM were introduced, the resulting suspension was stirred at RT for 5 min. then cooled down to 0-5°C using an ice- bath. 8.540 g of HATU (26.348 mmol, 3.0 eq.) were added at 0-5°C and the reaction mixture was stirred for 5 min at this temperature. Then 1.1 mL of 2-(2-aminoethyl)pyridine (8.783 mmol, 1.0 eq.) and 10 mL of DCM (due to the amount of HATU the suspension was too thick) were introduced at 0-5°C and the reaction mixture was stirred for 5 min at this temperature. Finally, 7.6 mL of DIPEA (43.914 mmol, 5.0 eq.) were added dropwise at 0-5°C. The resulting strong yellow mixture was stirred for 5 min at 0-5°C then the ice-bath was removed and the mixture was stirred O.N. at RT.

The reaction mixture, a brown-orange solution with a white solid, was filtered over a P3 sintered glass filter to remove the salt. The filtrate was transferred into a separating funnel using 5 mL of DCM and washed with 50 mL of NaCl 5.8%, then AP was extracted once with 10 mL of DCM. This operation was repeated 2 more times, then OPs were combined and washed once with 50 mL of NaHCO3 10% and finally with 50 mL of NaCl 11.6%. OP was dried over Na2SO4, filtered over a P3 sintered glass filter and concentrated to dryness into the rotavapor (40°C, 850-70 mbar). The material was dried under high vacuum (RT, 10-3 mbar) for 2h30 to yield 8.462 g (raw yield: 287.2%) of a brown-orange oily material as 362- VRO052-01-001 crude1#1.

A first attempt of purification by CC was performed using 50 g of silica gel (column with ø = 3.2 cm and h = 16.9 cm) and elution followed using TLC using DCM with 1% Et3N as eluant; Rf = 0.51. The product was eluted with a gradient from DCM with 1% Et3N to DCM with 0.5% of MeOH and 1% Et3N, fractions containing the desired product were combined and concentrated to dryness into the rotavapor (40°C, 800-50 mbar). The material was dried under high vacuum (RT, 10-3 mbar) for 3h to yield 6.666 g (raw yield: 226.3%) of a yellowish oily material as 362-VRO052-01-001 CC1#1.

A second attempt of purification to remove TMU by CC was performed using 55 g of silica gel (column with ø = 3.2 cm and h = 16.9 cm) and elution followed using TLC using heptane-EtOAc (2:8) and 1% Et3N as eluant; Rf = 0.45. The product was eluted with a gradient from heptane-EtOAc (7:3) with 1% Et3N to heptane-EtOAc (1:9) with 1% Et3N, fractions con- taining the desired product were combined and concentrated to dryness into the rotavapor (40°C, 220-50 mbar). The material was dried under high vacuum (RT, 10-3 mbar) for 2h to yield 3.228 g (raw yield: 109.6%) of a yellowish oily material as 362-VRO052-01-001 CC2#1.

In order to get rid from TMU, the material recovered from the 2nd CC was re-extracted. The material was transferred into a separating funnel using 15 mL of EtOAc, then washed 4 times with 50 mL of NaHCO3 10%. OP was dried over Na2SO4, filtered over a P3 sintered glass filter and concentrated to dryness into the rotavapor (40°C, 220-40 mbar). The material was dried under high vacuum (RT, 10-3 mbar) for 3h to yield 2.416g (raw yield: 82.0%) of a yellowish oily material as 362- VRO052-01-001 crude2#1.

¹H-NMR (300 MHz, CDCl3) δ = 8.52 (dt, J=4.8, 1.5, 1H, 16), 7.64 (td, J=7.7, 1.9, 1H, 14), 7.23 - 7.12 (m, 2H, 13, 15), 7.02 (br. s, 1H, 10), 5.09 - 4.77 (m, 1H, 4), 4.18 - 3.92 (m, 1H, 5), 3.78 - 3.56 (m, 2H, 11), 3.11 - 2.91 (m, 2H, 12), 1.61 (dd, J=16.0, 5.9, 2H, 6", 7), 1.45 (d, J=7.4, 1H, 6'), 1.40 (s, 9H, 1, 2, 3), 0.90 (d, J=6.1, 6H, 8, 9).

¹³C NMR (75 MHz, CDCl3) δ = 148.61 (16), 137.04 (14), 123.65 (13, 15), 121.68 (13, 15), 53.11 (5), 41.77 (6), 38.50 (11), 36.44 (12), 28.25 (1, 2, 3), 24.70 (7), 22.79 (8, 9), 21.99 (8, 9).

Then the Boc protecting group was removed as follows. In a 50 mL round-bottomed flask, equipped with a magnetic stirrer, 2.416 g of 362-VRO052-01-001 CC1#1 (7.202 mmol, eq.) and 18.1 mL of DCM were introduced, the resulting colourless solution was stirred at RT for 15 min then cooled down to 0-5°C using an ice-bath. 6.0 mL of TFA (78.875 mmol, 10.95 eq.) were added dropwise at 0-5°C. The reaction mixture was stirred for 5 min. at 0-5°C, then the ice-bath was removed and the mixture was stirred for 4h at RT. The reaction mixture was transferred into a separating funnel using 5 mL of DCM and washed with 50 mL of water. AP was basified using 20 mL of NaOH 25% to reach pH > 10, then extracted 3 times with 20 mL of DCM. OPs were combined and washed using 50 mL of NaCl 11.6%. OP was dried over Na₂SO₄, filtered over a P3 sintered glass filter and concen- trated to dryness into the rotavapor (40°C, 800-70 mbar). The material was dried under high vacuum (RT, 10-3 mbar) for 6h to yield 1.470 g (raw yield: 86.7%) of a pale yellow solid as 362-VRO052-02-001 crude1#1. This material was used "as is" without further purification in the next step.

¹H-NMR (300 MHz, CDCl₃) δ = 8.54 (d, J=4.5, 1H, 13), 7.70 (br. s, 1H, 7), 7.60 (td, J=7.7, 1.8, 1H, 11), 7.21 - 7.08 (m, 2H, 10, 12), 3.66 (q, J=6.4, 2H, 8), 3.38 (d, J=6.1, 1H, 2), 3.00 (t, J=6.6, 2H, 9), 1.74 (br. s, 2H, 1), 1.76 - 1.51 (m, 2H, 3", 4), 1.42 - 1.22 (m, 1H, 3'), 0.91 (dd, J=8.5, 6.2, 6H, 5, 6).

Finally, the dipeptide was coupled to the epoxy warhead. In a 100 mL round-bottomed flask, equipped with a magnetic stirrer, 970 mg of 362-BB01-03-004 crude1#1 (6.055 mmol, 1.05 eq.) and 15 mL of DMF were introduced, the resulting solution was stirred at RT for 5 min. then cooled down to 0- 5°C using an ice-bath. 5.607 g of HATU (17.299 mmol, 3.0 eq.) were added at 0-5°C and the reaction mixture was stirred for 5 min at this temperature. Then 1.357 g of 362-VRO052-02-001 crude1#1 (5.766 mmol, 1.0 eq.) in solution in 15 mL of DMF were introduced at 0-5°C and the reaction mixture was stirred for 5 min at this temperature. Finally, 5.0 mL of DIPEA (28.832 mmol, 5.0 eq.) were added dropwise at 0-5°C. The resulting yellow-brownish mixture was stirred for 5 min at 0-5°C then the ice-bath was removed and the mixture was stirred O.N. at RT.

The reaction mixture, a clear dark brown solution, was transferred into a separating funnel using 30 mL of EtOAc and washed with 100 mL of NaCl 5.8%, then AP was extracted 4 times with 30 mL of EtOAc. OPs were combined, and washed once with 100 mL NaHCO₃ 5%, then AP was extracted once with 20 mL of EtOAc. OPs were combined and washed once with 100 mL NaHCO₃ 10%, then AP was extracted once with 20 mL of EtOAc. OPs were combined and the last operation was repeated one more time. OP was dried over Na₂SO₄, filtered over a P3 sintered glass filter and concentrated to dryness into the rotavapor (40°C, 220-30 mbar). The material was dried under high vacuum (RT, 10-3 mbar) for 1h30 to yield 2.296 g (raw yield: 105.5%) of a dark brown sticky foam as 362-VRO052-03-002 crude1#1.

The crude material was firstly purified through a slurry using 20 mL of EtOAc. The suspension was stirred at RT for around 30 min, then cooled down to 0-5°C and filtered over a P3 sintered glass filter. The filter cake was washed twice with 6 mL of cold EtOAc, then dried under high vacuum (RT, 10-3 mbar) for 2h to yield 1.114 g (raw yield: 51.2%%) of a slightly beige powder as 362-VRO052-03-002 slurry1#1.

This material was re-purified by CC using 50 g of silica gel (column with ø = 3.2 cm and h = 16.9 cm) and elution followed using TLC using pure EtOAc with 1% Et₃N as eluant; R_{f} = 0.52. The product was eluted with a gradient from heptane- EtOAc (1:1) to pure EtOAc always with 1% Et₃N, fractions containing the desired product were combined and concentrated to dryness into the rotavapor (40°C, 240-50 mbar). The material was dried under high vacuum (RT, 10-3 mbar) for 3h to yield 966 mg (raw yield: 44.4%) of a white solid as 362-VRO052-03-002 CC1#1.

¹H-NMR (300 MHz, CDCl3) δ = 8.55 (d, J=4.9, 1H, 17), 7.69 (t, J=7.6, 1H, 16), 7.25 - 7.12 (m, 3H, 11, 14, 15), 6.60 (d, J=8.5, 1H, 5), 4.45 - 4.33 (m, 1H, 6), 4.27 (qd, J=7.2, 3.2, 2H, 2), 3.70 (t, J=6.3, 2H, 12), 3.66 (d, J=1.9, 1H, 4), 3.46 (d, J=1.9, 1H, 3), 3.03 (t, J=6.1, 2H, 13), 1.53 (br. m, 3H, 7, 8), 1.32 (t, J=7.1, 3H, 1), 0.89 (d, J=5.8, 6H, 9, 10).

¹³C-NMR (75 MHz, CDCl3) δ = 147.39 (17), 138.40 (15), 124.33 (14, 16), 122.27 (14, 16), 62.26 (2), 53.86 (4), 52.88 (3), 51.38 (6), 41.57 (7), 38.56 (12), 35.49 (13), 24.82 (8), 22.74 (9, 10), 22.01 (9, 10), 14.00 (1).

### Example 4: Synthesis of VRO059

Firstly, the dipeptide was formed. In a 50 mL round-bottomed flask, equipped with a magnetic stirrer, 1.320 g of Boc-L-Leu (5.707 mmol, 1.1 eq.) and 25 mL of DCM were introduced, the resulting turbid solution was stirred at RT for 5 min. then cooled down to 0-5°C using an ice- bath. 5.045 g of HATU (15.565 mmol, 3.0 eq.) were added at 0-5°C and the reaction mixture was stirred for 5 min at this temperature. Then 825 µL of 3-(piperidinyl)propanamine (5.188 mmol, 1.0 eq.) were introduced at 0-5°C and the reaction mixture was stirred for 5 min at this temperature. Finally, 4.5 mL of DIPEA (25.941 mmol, 5.0 eq.) were added dropwise at 0-5°C. The resulting yellowish mixture was stirred for 5 min at 0-5°C then the ice-bath was removed and the mixture was stirred O.N. at RT.

The reaction mixture, a brown-red solution with a white solid in suspension, was filtered over a P3 sintered glass filter to remove the salts. The filtrate was transferred into a separating funnel using 5 mL of DCM and washed with 50 mL of NaCl 5.8%, then AP was extracted once with 10 mL of DCM. OPs were combined and the previous washing were repeated 2 times. OPs were combined and washed once with 50 mL of NaHCO3 10% and finally with 50 mL of NaCl 11.6%. OP was dried over Na2SO4, filtered over a P3 sintered glass filter and concentrated to dryness into the rotavapor (40°C, 850-70 mbar). The material was dried under high vacuum (RT, 10-3 mbar) for 2h30 to yield 4.113 g (raw yield: 223.0%) of a brown- red oily material as 362-VRO059-01-002 crude1#1.

The crude material was purified by CC using 50 g of silica gel (column with ø = 3.2 cm and h = 16.9 cm) and elution followed using TLC using DCM with 1.5% of MeOH and 1% Et3N as eluant; Rf = 0.56. The product was eluted with a gradient from pure DCM to DCM with 2% MeOH always with 1% Et3N, fractions containing the desired product were combined and concentrated to dryness into the rotavapor (40°C, 750-60 mbar). The material was dried under high vacuum (RT, 10-3 mbar) for 2h to yield 1.557 g (raw yield: 84.4%) of a yellowish oil as 362-VRO059-01-002 CC1#1.

In order to get rid of TMU, the material recovered from the CC was re-extracted. The material was transferred into a separating funnel using 10 mL of EtOAc, then washed 2 times with 50 mL of NaHCO3 10%. OP was dried over Na2SO4, filtered over a P3 sintered glass filter and concentrated to dryness into the rotavapor (40°C, 220-40 mbar). The material was dried under high vacuum (RT, 10-3 mbar) for 3h to yield 1.181 g (raw yield: 64.0%) of a yellowish viscous material as 362-VRO059-01-001 crude2#1.

The re-extracted material was purified by a 2nd CC using 50 g of silica gel (column with ø = 3.2 cm and h = 16.9 cm) and elution followed using TLC using pure EtOAc with 1% of Et3N as eluant; Rf = 0.47. The product was eluted with a gradient from heptane-EtOAc (1:1) to pure EtOAc, fractions containing the desired product were combined and concentrated to dryness into the rotavapor (40°C, 220-60 mbar). The material was dried under high vacuum (RT, 10-3 mbar) for 2h to yield 1.038 g (raw yield: 56.3%) of a pale yellowish solid as 362-VRO059-01-002 CC2#1.

¹H-NMR (300 MHz, CDCl3) δ = 7.63 (br. s, 1H, 10), 4.98 (d, J=7.7, 1H, 4), 4.10 (br. s, 1H, 5), 3.46 - 3.21 (m, 2H, 11), 2.71 - 2.13 (m, 6H, 13, 14, 15), 1.79 - 1.55 (m, 8H, 6", 7, 12, 16, 17), 1.54 - 1.35 (m, 3H, 6', 18), 1.43 (s, 9H, 1, 2, 3), 0.94 (dd, J=6.2, 3.5, 6H, 8, 9).

¹³C NMR (75 MHz, CDCl3) δ = 58.15 (13), 54.56 (14, 15), 53.15 (5), 42.32 (6), 39.62 (11), 28.27 (1, 2, 3), 25.84 (12, 16, 17), 24.66 (7), 24.61 (12, 16, 17), 24.11 (18), 23.05 (8, 9), 21.97 (8, 9).

Then the Boc protecting group was removed as follows. In a 25 mL round-bottomed flask, equipped with a magnetic stirrer, 1.024 g of 362-VRO059-01-001 CC2#1 (2.880 mmol, eq.) and 7.7 mL of DCM were introduced, the resulting colourless solution was stirred at RT for 10 min then cooled down to 0-5°C using an ice-bath. 2.6 mL of TFA (33.430 mmol, 11.61 eq.) were added dropwise at 0-5°C. The reaction mixture was stirred for 5 min. at 0-5°C, then the ice-bath was removed and the mixture was stirred for 4h at RT. The reaction mixture was transferred into a separating funnel using 5 mL of DCM and washed with 50 mL of water. AP was basified using 8 mL of NaOH 25% to reach pH > 10, then extracted 3 times with 20 mL of DCM. OPs were combined and washed using 50 mL of NaCl 11.6%. OP was dried over Na₂SO₄, filtered over a P3 sintered glass filter and concentrated to dryness into the rotavapor (40°C, 800-70 mbar). The material was dried under high vacuum (RT, 10-3 mbar) for 6h to yield 654 mg (raw yield: 88.9%) of a pale yellowish oil as 362-VRO059-02-002 crude1#1. This material was used "as it" without further purification in the next step.

¹H-NMR (300 MHz, CDCl₃) δ = 7.90 (br. s, 1H, 7), 3.44 - 3.21 (m, 3H, 2, 8), 2.61 - 2.26 (m, 6H, 10, 11, 12), 1.66 (br. m, 10H, 1, 3', 4, 9', 9", 13, 14), 1.51 - 1.41 (m, 2H, 15), 1.40 - 1.27 (m, 1H, 3"), 0.94 (dd, J=8.0, 6.5, 6H, 5, 6).

¹³C-NMR (75 MHz, CDCl₃) δ = 57.78 (10), 54.58 (11, 12), 53.74 (2), 44.32 (3), 38.64 (8), 25.75 (9, 13, 14), 25.59 (9, 13, 14), 24.80 (4), 24.18 (15), 23.39 (5, 6), 21.37 (5, 6).

Finally, the dipeptide was coupled to the epoxy warhead. In a 25 mL round-bottomed flask, equipped with a magnetic stirrer, 356 mg of 362-BB01-03-004 crude1#1 (2.241 mmol, 1.05 eq.) and 7.5 mL of DMF were introduced, the resulting solution was stirred at RT for 5 min. then cooled down to 0- 5°C using an ice-bath. 2.075 g of HATU (6.402 mmol, 3.0 eq.) were added at 0-5°C and the reaction mixture was stirred for 5 min at this temperature. Then 545 mg of 362-VRO059-02-002 crude1#1 (2.134 mmol, 1.0 eq.) in solution in 7.5 mL of DMF were introduced at 0-5°C and the reaction mixture was stirred for 5 min at this temperature. Finally, 1.9 mL of DIPEA (10.699 mmol, 5.0 eq.) were added dropwise at 0-5°C. The resulting yellow-brownish mixture was stirred for 5 min at 0-5°C then the ice-bath was removed and the mixture was stirred O.N. at RT.

The reaction mixture, a clear dark brown solution, was transferred into a separating funnel using 15 mL of EtOAc and washed with 50 mL of NaCl 5.8%, then AP is extracted 4 times with 15 mL of EtOAc. OPs are combined and washed 4 times with 50 mL NaHCO₃ 10%. OP was dried over Na₂SO₄, filtered over a P3 sintered glass filter and concentrated to dryness into the rotavapor (40°C, 220-30 mbar). The material was dried under high vacuum (RT, 10-3 mbar) for 1h30 to yield 1.063 g (raw yield: 125.3%) of a dark brown sticky solid as 362-VRO059-03-003 crude1#1.

The crude material was purified by CC using 50 g of silica gel (column with ø = 3.2 cm and h = 16.9 cm) and elution followed using TLC using DCM containing 1% MeOH and 1% Et₃N as eluant; R_{f} = 0.36. The product was eluted with a gradient from pure DCM to DCM with 1% MeOH always with 1% Et₃N, fractions containing the desired product were combined and concentrated to dryness into the rotavapor (40°C, 800-50 mbar). The material was dried under high vacuum (RT, 10-3 mbar) for 2h to yield 397 mg (raw yield: 46.8%) of a viscous brown-orange solid as 362-VRO059-03-003 CC1#1.

This material was re-purified by CC using 50 g of silica gel (column with ø = 3.2 cm and h = 16.9 cm) and elution followed using TLC using heptane-acetone (3:7) with 1% Et₃N as eluant; R_{f} = 0.57. The product was eluted with a gradient from heptane-acetone (8:2) to heptane-acetone (3:7) always with 1% Et₃N, fractions containing the desired product were combined and concentrated to dryness into the rotavapor (40°C, 320-50 mbar). The material was dried under high vacuum (RT, 10-3 mbar) for 1h to yield 116 mg (raw yield: 17.4%) of a brown-beige solid as 362-VRO059-03-003 CC2#1.
This material was re-extracted; it was transferred into a separating funnel using 20 mL of EtOAc and 20 mL of water, then HCl 1M was added to reach pH 1. AP was the basified to pH 9 and extracted 3 times with 20 mL of EtOAc. OPs were combined and was dried over Na₂SO₄, filtered over a P3 sintered glass filter and concentrated to dryness into the rotavapor (40°C, 220-30 mbar). The material was dried under high vacuum (RT, 10-3 mbar) for 3h to yield 111 mg (raw yield: 13.7%) of a viscous orange-yellow solid as 362-VRO059-03-003 extrac1#1.

¹H-NMR (300 MHz, CDCl₃) δ = 8.17 (t, J=5.0, 1H, 11), 6.69 (d, J=8.5, 1H, 5), 4.43 - 4.31 (m, 1H, 6), 4.25 (qq, J=7.1, 3.6, 2H, 2), 3.67 (d, J=1.9, 1H, 4), 3.46 (d, J=1.9, 1H, 3), 3.44 - 3.23 (m, 2H, 12), 2.73 - 2.30 (m, 6H, 14, 15, 16), 1.63 (br. m, 11H, 7, 8, 13, 17, 18, 19), 1.30 (t, J=7.1, 3H, 1), 0.93 (d, J=5.3, 6H, 9, 10).

¹³C-NMR (75 MHz, CDCl₃) δ = 62.20 (2), 58.25 (14), 54.47 (15, 16), 53.89 (4), 52.84 (3), 51.43 (6), 42.09 (7), 40.01 (12), 25.79 (13, 17, 18, 19), 24.76 (8), 23.98 (13, 17, 18, 19), 23.87 (13, 17, 18, 19), 22.95 (9, 10), 22.02 (9, 10), 13.99 (1).

### Example 5: Synthesis of VRO073

Firstly, the dipeptide was formed. In a 25 mL round-bottomed flask, equipped with a magnetic stirrer, 970 mg of Boc-L-Leu (4.195 mmol, 1.05 eq.) and 10 mL of DCM were introduced, the resulting turbid solution was stirred at RT for 5 min. then cooled down to 0-5°C using an ice-bath. 1.943 g of HATU (5.993 mmol, 1.5 eq.) were added at 0-5°C and the reaction mixture was stirred for 5 min at this temperature. Then 457 µL of 2-fluorobenzylamine (3.995 mmol, 1.0 eq.) were introduced at 0-5°C and the reaction mixture was stirred for 5 min at this temperature. Finally, 1.4 mL of DIPEA (7.991 mmol, 2.0 eq.) were added dropwise at 0-5°C. The resulting turbid and strong yellow solution was stirred for 5 min at 0-5°C then the ice-bath was removed and the mixture was stirred O.N. at RT.

The reaction mixture, a slightly turbid yellow solution, was transferred into a separating funnel using 10 mL of DCM and washed with 50 mL of NaCl 5.8%, then AP was extracted once with 10 mL of DCM. This operation was repeated 2 more times, then OPs were combined and washed once with 50 mL of NaHCO3 10% and finally with 50 mL of NaCl 11.6%. OP was dried over Na2SO4, filtered over a P3 sintered glass filter and concentrated to dryness into the rotavapor (40°C, 850- 70 mbar). The material was dried under high vacuum (RT, 10-3 mbar) for 3h to yield 1.841 g (raw yield: 136.2%) of a beige- yellow viscous solid as 362-VRO073-01-001 crude1#1.

The crude material was purified by CC using 45 g of silica gel (column with ø = 3.2 cm and h = 15.2 cm) and elution followed using TLC using heptane-EtOAc (7:3) as eluant; Rf = 0.49. The product was eluted with a gradient from pure heptane to heptane-EtOAc (6:4), fractions containing the desired product were combined and concentrated to dryness into the rotavapor (40°C, 220-50 mbar). The material was dried under high vacuum (RT, 10-3 mbar) for 2h to yield 1.261 g (raw yield: 93.3%) of a white foam as 362-VRO073-01-001 CC1#1.

¹H-NMR (300 MHz, CDCl3) δ = 7.31 (td, J=7.8, 1.6, 1H, 14), 7.27 - 7.18 (m, 1H, 12), 7.14 - 6.96 (m, 2H, 13, 15), 6.54 (br. s, 1H, 10), 4.85 (s, 1H, 4), 4.85 (br. s, J=6.8, 1H), 4.48 (d, J=4.6, 2H, 11), 4.10 (br. s, 1H, 5), 1.77 - 1.57 (m, 2H, 6', 7), 1.55 - 1.45 (m, 1H, 6"), 1.41 (s, 9H, 1, 2, 3), 0.92 (dd, J=6.3, 2.9, 6H, 8, 9).

¹³C NMR (75 MHz, CDCl3) δ = 129.95 (12, 14), 129.23 (12, 14), 129.12 (12, 14), 124.24 (13), 124.19 (13), 115.41 (15), 115.13 (15), 53.04 (5), 40.90 (6), 37.40 (11), 37.34 (11), 28.18 (1, 2, 3), 24.68 (7), 22.85 (8, 9), 21.92 (8, 9).

Then the Boc protecting group was removed as follows. In a 25 mL round-bottomed flask, equipped with a magnetic stirrer, 1.249 g of 362-VRO073-01-001 CC1#1 (3.691 mmol, 1.0 eq.) and 9.4 mL of DCM were introduced, the resulting colourless solution was stirred at RT for 10 min then cooled down to 0-5°C using an ice-bath. 3.1 mL of TFA (40.48 mmol, 10.97 eq.) were added dropwise at 0-5°C. The reaction mixture was stirred for 5 min. at 0-5°C, then the ice-bath was removed and the mixture was stirred for 2h30 at RT. The reaction mixture was transferred into a separating funnel using 25 mL of DCM and washed with 25 mL of water. AP was basified using 3 mL of NaOH 25% to reach pH > 10, then extracted 3 times with 25 mL of DCM. OPs were combined and washed using 25 mL of NaCl 11.6%. OP was dried over Na₂SO₄, filtered over a P3 sintered glass filter and concentrated to dryness into the rotavapor (40°C, 800-70 mbar). The material was dried under high vacuum (RT, 10-3 mbar) for 1h to yield 465 mg (raw yield: 52.9%) of an almost colourless oil as 362-VRO073-02-001 crude1#1. This material was used "as it" without further purification in the next step.

¹H-NMR (300 MHz, CDCl₃) δ = 7.67 (br. s, 1H, 7), 7.32 (td, J=7.7, 1.7, 1H, 11), 7.29 - 7.18 (m, 1H, 9), 7.15 - 6.98 (m, 2H, 10, 12), 4.49 (d, J=6.1, 2H, 8), 3.42 (dd, J=9.9, 3.4, 1H, 2), 1.83 - 1.62 (m, 2H, 3", 4), 1.57 - 1.21 (m, 3H, 1, 3'), 0.94 (dd, J=8.7, 6.0, 6H, 5, 6).

Finally, the dipeptide was coupled to the epoxy warhead. In a 25 mL round-bottomed flask, equipped with a magnetic stirrer, 320 mg of 362-BB01-03-001 crude1#1 (1.996 mmol, 1.05 eq.) and 5 mL of DCM were introduced, the resulting white suspension was stirred at RT for 5 min. then cooled down to 0-5°C using an ice-bath. 924 mg of HATU (2.851 mmol, 1.5 eq.) were added at 0-5°C and the reaction mixture was stirred for 5 min at this temperature. Then 453 mg of 362-VRO073-02-001 crude1#1 (1.901 mmol, 1.0 eq.) in solution in 5 mL of DCM were introduced at 0-5°C and the reaction mixture was stirred for 5 min at this temperature. Finally, 993 µL of DIPEA (5.703 mmol, 3.0 eq.) were added dropwise at 0-5°C. The resulting yellowish mixture was stirred for 5 min at 0- 5°C then the ice-bath was removed and the mixture was stirred O.N. at RT.

The reaction mixture, a yellow-orange solution, was transferred into a separating funnel using 10 mL of DCM and washed with 50 mL of NaCl 2.6%, then AP was extracted once with 10 mL of DCM. OPs were combined, and washed once with 50 mL NaCl 2.6%, then AP was extracted once with 10 mL of DCM. OPs were combined and washed with 50 mL of NaCl 5.8% and 5 mL of HCl 1M, which resulted in an emulsion that had to be filtered on celite. The filtrate was transferred again into the separating funnel using 10 mL of DCM and washed once with 50 mL of NaHCO₃ 10% and once with 50 mL of NaCl 11.6%. OP was dried over Na₂SO₄, filtered over a P3 sintered glass filter and concentrated to dryness into the rotavapor (40°C, 220-30 mbar). The material was dried under high vacuum (RT, 10-3 mbar) for 2h to yield 835 mg (raw yield: 115.5%) of a pasty orange solid as 362-VRO073-03-001 crude1#1.

The crude material was purified by CC using 45 g of silica gel (column with ø = 3.2 cm and h = 15.2 cm) and elution followed using TLC using heptane-EtOAc (1:1) as eluant; R_{f} = 0.50. The product was eluted with a gradient from pure heptane to heptane-EtOAc (2:8), fractions containing the desired product were combined and concentrated to dryness into the rotavapor (40°C, 200-50 mbar). The material was dried under high vacuum (RT, 10-3 mbar) for 3h to yield 110 mg (raw yield: 15.2%) of a white solid as 362-VRO073-03-001 CC1#1.

¹H-NMR (300 MHz, CDCl₃) δ = 7.36 - 7.21 (m, 2H, 13, 15), 7.17 - 6.99 (m, 2H, 14, 16), 6.51 (d, J=8.6, 1H, 5), 6.35 (t, J=5.5, 1H, 11), 4.48 (dd, J=5.8, 2.5, 2H, 12), 4.41 (td, J=8.5, 5.8, 1H, 6), 4.27 (qq, J=6.9, 3.6, 2H, 2), 3.66 (d, J=1.9, 1H, 4), 3.44 (d, J=1.9, 1H, 3), 1.75 - 1.60 (m, 1H, 7"), 1.60 - 1.44 (m, 2H, 7', 8), 1.31 (t, J=7.1, 3H, 1), 0.90 (dd, J=6.2, 4.5, 6H, 9, 10).

### Example 6: Synthesis of VRO109

Firstly, the dipeptide was formed. In a 25 mL round-bottomed flask, equipped with a magnetic stirrer, 190 mg of (S)-Boc-2-amino-4,4,4-trifluoro-butyric acid (0.739 mmol, 1.0 eq.) and 10 mL of DCM were introduced, the resulting mixture was stirred at RT for 5 min. then cooled down to 0-5°C using an ice-bath. 359 mg of HATU (1.108 mmol, 1.5 eq.) were added at 0-5°C and the reaction mixture was stirred for 5 min at this temperature. Then 94 µL of IAA (0.813 mmol, 1.1 eq.) were introduced at 0-5°C and the reaction mixture was stirred for 5 min at this temperature. Finally, 257 µL of DIPEA (1.477 mmol, 2.0 eq.) were added dropwise at 0-5°C. The resulting slightly pale yellow mixture was stirred for 5 min at 0-5°C then the ice-bath was removed and the mixture was stirred O.N. at RT.

The reaction mixture, a yellow-orange solution, was concentrated to dryness into the rotavapor (40°C, 750-400 mbar). The residue was transferred into a separating funnel using 20 mL of EtOAc and washed with 50 mL of NaHCO3 5%, then AP was extracted 2 times with 15 mL of EtOAc. OPs were combined, and washed 2 times with 50 mL NaHCO3 5%, once with 50 mL of NaHCO3 10% and once with 50 mL of NaCl 11.6%. OP was dried over Na2SO4, filtered over a P3 sintered glass filter and concentrated to dryness into the rotavapor (40°C, 220-30 mbar). The material was dried under high vacuum (RT, 10-3 mbar) for 6h to yield 224 mg (raw yield: 92.9%) of an off-white crystallized solid as 362-VRO109-01-001 crude1#1.

The crude material was purified by CC using 50 g of silica gel (column with ø = 3.2 cm and h = 16.9 cm) and elution followed using TLC using heptane-EtOAc (7:3) as eluant; Rf = 0.46. The product was eluted with a gradient from pure heptane to heptane-EtOAc (6:4), fractions containing the desired product were combined and concentrated to dryness into the rotavapor (40°C, 220-50 mbar). The material was dried under high vacuum (RT, 10-3 mbar) for 4h to yield 204 mg (raw yield: 84.6%) of a white foam as 362-109-01-001 CC1#1.

¹H-NMR (300 MHz, CDCl3) δ = 6.26 (br. s, 1H, 7), 4.97 (d, J=6.6, 1H, 4), 4.49 - 4.26 (m, 1H, 5), 3.39 - 3.16 (m, 2H, 8), 2.93 - 2.69 (m, 1H, 6'), 2.62 - 2.37 (m, 1H, 6"), 1.69 - 1.51 (m, 1H, 10), 1.45 (s, 9H, 1, 2, 3), 1.39 (q, J=7.3, 2H, 9), 0.91 (d, J=6.6, 6H, 11, 12).

¹³C NMR (75 MHz, CDCl3) δ = 49.48 (5), 38.12 (9), 38.00 (8), 28.15 (1, 2, 3), 25.67 (10), 22.32 (11, 12).

Then the Boc protecting group was removed as follows. In a 10 mL round-bottomed flask, equipped with a magnetic stirrer, 201 mg of 362-VRO109-01-001 CC1#1 (0.616 mmol, eq.) and 5 mL of DCM were introduced, the resulting colourless solution was stirred at RT for 10 min then cooled down to 0-5°C using an ice-bath. 1.7 mL of TFA (22.200 mmol, 36.045 eq.) were added dropwise at 0-5°C. The reaction mixture was stirred for 5 min. at 0-5°C, then the ice-bath was removed and the mixture was stirred for 1h at RT. The reaction mixture was transferred into a separating funnel using 25 mL of DCM and washed with 50 mL of water. AP was basified using 4 mL of NaOH 25% to reach pH > 10, then extracted 3 times with 15 mL of DCM. OPs were combined and dried over Na₂SO₄, filtered over a P3 sintered glass filter and concentrated to dryness into the rotavapor (40°C, 750-400 mbar). The material was dried under high vacuum (RT, 10-3 mbar) for 6h to yield 256 mg (raw yield: 96.6%) of an almost colourless oil as 362-VRO109-02-001 crude1#1. This material was used "as it" without further purification in the next step.

¹H-NMR (300 MHz, DMSO-d6-d6) δ = 8.07 (t, J=5.3, 1H, 4), 3.48 (dd, J=7.5, 5.4, 1H, 2), 3.19 - 2.97 (m, 2H, 5), 2.78 - 2.53 (m, 1H, 3"), 2.49 - 2.24 (m, 1H, 3'), 1.56 (hept, J=6.7, 1H, 7), 1.30 (q, J=7.2, 2H, 6), 0.86 (dd, J=6.6, 0.6, 6H, 8, 9).

¹³C-NMR (75 MHz, DMSO-d6) δ = 49.20 (2), 49.16 (2), 37.62 (6), 37.46 (3), 37.11 (3), 36.57 (5), 24.78 (7), 22.07 (8, 9), 22.05 (8, 9).

Finally, the dipeptide was coupled to the epoxy warhead. In a 25 mL round-bottomed flask, equipped with a magnetic stirrer, 115 mg of 362-BB01-03-004 crude1#1 (0.720 mmol, eq.) and 5 mL of DMF were introduced, the resulting solution was stirred at RT for 5 min. then cooled down to 0-5°C using an ice-bath. 318 mg of HATU (0.981 mmol, 1.5 eq.) were added at 0-5°C and the reaction mixture was stirred for 5 min at this temperature. Then 148 mg of 362-VRO109-02-001 crude1#1 (0.654 mmol, 1.0 eq.) in solution in 5 mL of DMF were introduced at 0-5°C and the reaction mixture was stirred for 5 min at this temperature. Finally, 228 µL of DIPEA (1.308 mmol, 2.0 eq.) were added dropwise at 0-5°C. The resulting yellow mixture was stirred for 5 min at 0-5°C then the ice-bath was removed and the mixture was stirred O.N. at RT.

The reaction mixture, an orange solution, was s transferred into a separating funnel using 20 mL of EtOAc and washed with 50 mL of NaHCO₃ 5%, then AP was extracted 2 times with 15 mL of EtOAc. OPs were combined, and washed 3 times with 50 mL NaHCO₃ 5% and once with 50 mL of NaCl 11.6%. OP was dried over Na₂SO₄, filtered over a P3 sintered glass filter and concentrated to dryness into the rotavapor (40°C, 220-140 mbar). The material was dried under high vacuum (RT, 10-3 mbar) for 6h to yield 237 mg (raw yield: 102.5%) of a very pale orange solid as 362-VRO109-03-001 crude1#1.

The crude material was purified through a slurry using 5 mL of EtOAc-heptane (1:1). The suspension was stirred at RT for 20 min then cooled down to 0-5°C and stirred for 15 min. The suspension was filtered over an ultrafiltration unit equipped with a nylon membrane (0.45 µm), the filter cake was washed 3 times with cold EtOAc-heptane (1:9), then dried under high vacuum (RT, 10-3 mbar) for 3h to yield 175 mg (raw yield: 72.6%) of a white powder as 362-VRO109-03-001 slurry1#1.

¹H-NMR (300 MHz, CDCl₃) δ = 6.59 (d, J=8.6, 1H, 5), 6.18 (s, 1H, 8), 4.70 (td, J=8.9, 4.8, 1H, 6), 4.28 (qd, J=7.1, 2.5, 2H, 2), 3.72 (d, J=1.9, 1H, 4), 3.45 (d, J=1.8, 1H, 3), 3.27 (tdd, J=7.4, 5.8, 4.7, 2H, 9), 2.74 (ddt, J=16.4, 10.7, 5.3, 1H, 7'), 2.65 - 2.40 (m, 1H, 7"), 1.60 (dt, J=13.4, 7.0, 2H, 11), 1.40 (q, J=7.2, 2H, 10), 1.32 (t, J=7.2, 3H, 1), 1.25 (d, J=1.8, 0H), 0.91 (d, J=6.6, 6H, 12, 13).

### Example 7: Synthesis of VRO244

Firstly, the dipeptide was formed. In a 25 mL round-bottomed flask, equipped with a magnetic stirrer, 190 mg of Boc-(S)-2-amino-4,4,4-butyric acid (0.739 mmol, 1.0 eq.) and 10 mL of DCM were introduced, the resulting mixture was stirred at RT for 5 min. then cooled down to 0-5°C using an ice-bath. 359 mg of HATU (1.108 mmol, 1.5 eq.) were added at 0-5°C and the reaction mixture was stirred for 5 min at this temperature. Then 93 µL of 2-fluorobenzylamine (0.813 mmol, 1.1 eq.) were introduced at 0-5°C and the reaction mixture was stirred for 5 min at this temperature. Finally, 257 µL of DIPEA (1.477 mmol, 2.0 eq.) were added dropwise at 0-5°C. The resulting slightly pale yellow mixture was stirred for 5 min at 0-5°C then the ice-bath was removed and the mixture was stirred O.N. at RT.

The reaction mixture, a clear yellow solution, was concentrated to dryness into the rotavapor (40°C, 750-400 mbar). The residue was transferred into a separating funnel using 20 mL of EtOAc and washed with 50 mL of NaHCO3 5%, then AP was extracted 2 times with 15 mL of EtOAc. OPs were combined, and washed 2 times with 50 mL NaHCO3 5%, once with 50 mL of NaHCO3 10% and once with 50 mL of NaCl 11.6%. OP was dried over Na2SO4, filtered over a P3 sintered glass filter and concentrated to dryness into the rotavapor (40°C, 220-140 mbar). The material was dried under high vacuum (RT, 10-3 mbar) for 6h to yield 279 mg (raw yield: 103.7%) of an orange solid as 362-VRO244-01-001 crude1#1.

The crude material was purified by CC using 50 g of silica gel (column with ø = 3.2 cm and h = 16.9 cm) and elution followed using TLC using heptane-acetone (7:3) as eluant; Rf = 0.45. The product was eluted with a gradient from pure heptane to heptane-acetone (6:4), fractions containing the desired product were combined and concentrated to dryness into the rotavapor (40°C, 350-50 mbar). The material was dried under high vacuum (RT, 10-3 mbar) for 2h to yield 259 mg (raw yield: 96.2%) of a slightly pale yellow solid as 362-VRO243-01-001 CC1#1.

¹H-NMR (300 MHz, CDCl3) δ = 7.36 - 7.20 (m, 2H, 9, 11), 7.14 - 7.06 (m, 1H, 10), 7.09 - 6.98 (m, 1H, 12), 6.73 (br. s, 1H, 7), 4.95 (br. s, 1H, 4), 4.61 - 4.31 (m, 3H, 5, 8), 2.96 - 2.72 (m, 1H, 6'), 2.65 - 2.38 (m, 1H, 6"), 1.42 (s, 9H, 1, 2, 3).

¹³C NMR (75 MHz, CDCl3) δ = 130.01 (9, 11), 129.53 (9, 11), 129.41 (9, 11), 124.33, (10), 124.28 (10), 115.54 (12), 115.26 (12), 49.56 (5), 37.81 (6, 8), 37.76 (6, 8), 28.10 (1, 2, 3).

Then the Boc protecting group was removed as follows. In a 10 mL round-bottomed flask, equipped with a magnetic stirrer, 252 mg of 362-VRO244-01-001 CC1#1 (0.692 mmol, 1.0 eq.) and 5 mL of DCM were introduced, the resulting colourless solution was stirred at RT for 10 min then cooled down to 0-5°C using an ice-bath. 1.7 mL of TFA (22.200 mmol, 32.10 eq.) were added dropwise at 0-5°C. The reaction mixture was stirred for 5 min. at 0-5°C, then the ice-bath was removed and the mixture was stirred for 1h at RT. The reaction mixture was transferred into a separating funnel using 25 mL of DCM and washed with 50 mL of water. AP was basified using 4 mL of NaOH 25% to reach pH > 10, then extracted 3 times with 15 mL of DCM. OPs were combined and dried over Na₂SO₄, filtered over a P3 sintered glass filter and concentrated to dryness into the rotavapor (40°C, 750-400 mbar). The material was dried under high vacuum (RT, 10-3 mbar) for 4h to yield 172 mg (raw yield: 94.1%) of an almost colourless oil as 362-VRO244-02-001 crude1#1. This material was used "as it" without further purification in the next step.

¹H-NMR (300 MHz, DMSO-d6) δ = 8.62 (t, J=4.7, 1H, 4), 7.39 - 7.23 (m, 2H, 6, 8), 7.22 - 7.05 (m, 2H, 7, 9), 4.34 (d, J=5.9, 2H, 5), 3.58 (dd, J=7.8, 5.1, 1H, 2), 3.03 (s, 2H, 1), 2.82 - 2.58 (m, 1H, 3"), 2.48 - 2.30 (m, 1H, 3').

¹³C-NMR (75 MHz, DMSO-d6) δ = 129.26 (8), 129.21 (8), 128.69 (6), 128.58 (6), 124.00 (7), 123.95 (7), 114.86 (9), 114.58 (9), 49.29 (2), 49.25 (2), 37.78 (3), 37.43 (3), 37.08 (3), 36.73 (3), 35.85 (5), 35.79 (5).

Finally, the dipeptide was coupled to the epoxy warhead. In a 25 mL round-bottomed flask, equipped with a magnetic stirrer, 111 mg of 362-BB01-03-005 crude1#1 (0.695 mmol, eq.) and 5 mL of DMF were introduced, the resulting DMF was stirred at RT for 5 min. then cooled down to 0-5°C using an ice-bath. 307 mg of HATU (0.948 mmol, 1.5 eq.) were added at 0-5°C and the reaction mixture was stirred for 5 min at this temperature. Then 167 mg of 362-VRO244-02-001 crude1#1 (0.632 mmol, 1.0 eq.) in solution in 5 mL of DMF were introduced at 0-5°C and the reaction mixture was stirred for 5 min at this temperature. Finally, 220 µL of DIPEA (1.264 mmol, 2.0 eq.) were added dropwise at 0-5°C. The resulting yellow mixture was stirred for 5 min at 0-5°C then the ice-bath was removed and the mixture was stirred O.N. at RT.

The reaction mixture, an orange solution, was transferred into a separating funnel using 20 mL of EtOAc and washed with 50 mL of NaHCO₃ 5%, then AP was extracted 2 times with 15 mL of EtOAc. OPs were combined, and washed 3 times with 50 mL NaHCO₃ 5% and once with 50 mL of NaCl 11.6%. OP was dried over Na₂SO₄, filtered over a P3 sintered glass filter and concentrated to dryness into the rotavapor (40°C, 220-30 mbar). The material was dried under high vacuum (RT, 10-3 mbar) for 2h to yield 274 mg (raw yield: 106.7%) of a brown-orange solid as 362-VRO244-03-001 crude1#1.

The crude material was purified through a slurry using 3 mL of EtOAc-heptane (2:1). The granular suspension was stirred at 40°C for 10 min, then at RT for 15 min and finally cooled down to 0-5°C using an ice-bath before filtration. The suspen- sion was filtered over an ultrafiltration unit equipped with a nylon membrane (0.45 µm), the filter cake was washed and triturated 5 times with 1 mL of cold EtOAc-heptane (1:1), then dried under high vacuum (RT, 10-3 mbar) for 2h to yield 131 mg (raw yield: 51.0%) of off-white granules as 362-VRO244-03-001 slurry1#1.

¹H-NMR (300 MHz, DMSO-d6) δ = 8.89 (d, J=8.6, 1H, 5), 8.76 (t, J=5.8, 1H, 8), 7.38 - 7.24 (m, 2H, 10, 12), 7.24 - 7.11 (m, 2H, 11, 13), 4.70 (td, J=9.0, 4.0, 1H, 6), 4.33 (d, J=5.8, 2H, 9), 4.27 - 4.10 (m, 2H, 2), 3.69 (d, J=1.8, 1H, 4), 3.57 (d, J=1.8, 1H, 3), 2.91 - 2.55 (br. m, 2H, 7), 1.23 (t, J=7.1, 3H, 1).

¹³C-NMR (75 MHz, DMSO-d6) δ = 129.08 (10, 12), 129.02 (10, 12), 128.76 (10, 12), 128.65 (10, 12), 124.02 (11), 114.92 (13), 114.64 (13), 61.37 (2), 52.87 (4), 51.09 (3), 46.83 (6), 35.99 (9), 35.93 (9), 34.39 (7), 34.05 (7), 13.63 (1).

### Exampe 8: In-vitro enzymatic activity assays on isolated enzymes

The ability of the compounds VRO006_hydrol, VRO047_hydrol, VRO052_hydrol, VRO059_hydrol, VRO073_hydrol, VRO109_hydrol, VRO244_hydrol, VRO001_hydrol, VRO082_hydrol, VRO035_hydrol, VRO119_hydrol, and VRO243_hydrol to inhibit isolated cathepsin B was assessed in an enzymatic assay and compared to the inhibitory activity of the prior art E64c. In this assay, the compounds VRO006, VRO042, VRO047, VRO052, VRO059, VRO073, VRO109, VRO244, VRO001, VRO082, VRO035, VRO119, and VRO243 were used and the assay was performed in the presence of NaOH, in order to simulate the ester cleavage that occurs within the gastrointestinal tract, and to release the free acid (VROxxx_hydrol compound) from the prodrug (corresponding VROxxx compound). The chemical structures of compounds VRO006, VRO042, VRO047, VRO052, VRO059, VRO073, VRO109 and VRO244 and their chemical names are provided in [Table 2] above. The structures of VRO001, VRO082, VRO035, VRO119 and VRO243 are provided in [Table 3] below. The cathepsin B was from pork liver homogenate.

**[Table 3]**

| Compound reference | Structure | Chemical name |
|---|---|---|
| VRO001 | 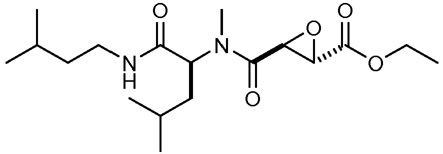 | Ethyl (2S,3S)-3-(((S)-1-(isopentylamino)-4-methyl-1-oxopentan-2-yl)(methyl)carbamoyl)oxirane-2-carboxylate |
| VRO082 | 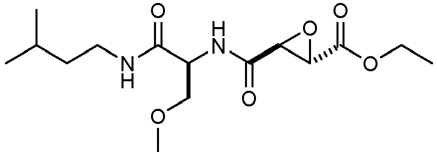 | Ethyl (2S,3S)-3-(((S)-1-(isopentylamino)-3-methoxy-1-oxopropan-2-yl)carbamoyl)oxirane-2-carboxylate |
| VRO035 | 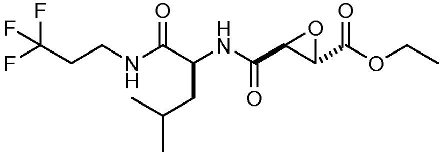 | Ethyl (2S,3S)-3-(((S)-4-methyl-1-oxo-1-((3,3,3-trifluoropropyl)amino)pentan-2-yl)carbamoyl)oxirane-2-carboxylate |
| VRO119 | 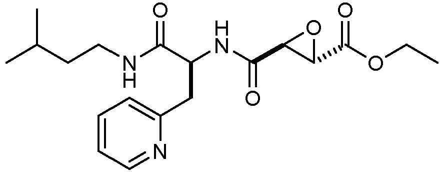 | Ethyl (2S,3S)-3-(((S)-1-(isopentylamino)-1-oxo-3-(pyridin-2-yl)propan-2-yl)carbamoyl)oxirane-2-carboxylate |
| VRO243 | 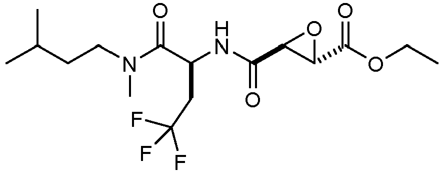 | Ethyl (2S,3S)-3-(((S)-4,4,4-trifluoro-1-(isopentyl(methyl)amino)-1-oxobutan-2-yl)carbamoyl)oxirane-2-carboxylate |

The enzymatic assay was performed as follows. Samples of homogenized pork liver (75 mg of fresh tissue per sample) were incubated for 1 hour at 37°C with Z-FR-AMC (benzyloxycarbonyl-L-arginyl-L-arginine-4-methylcoumaryl-7-amide) at 50 mM in 1 M sodium acetate pH 6.0, supplemented with 2.5 mM EDTA, 2.5 mM DTT and 0.1% Triton X-100, and graded concentrations of test substances (between 0.1 nM and 50 mM). The stock solutions of test substances were at 20 mM in DMSO. Fluorescence was determined at 445 nm using 365 nm as the excitation wavelength.

The compounds of the invention VRO006_hydrol, VRO047_hydrol, VRO052_hydrol, VRO059_hydrol, VRO073_hydrol, VRO109_hydrol and VRO244_hydrol exhibited a suitable inhibitory activity with an IC50 of about 100 nM or less (see [Fig. 1]), whereas the comparative compounds VRO001_hydrol, VRO082_hydrol, VRO035_hydrol, VRO119_hydrol and VRO243_hydrol did not efficiently inhibit catB, with an IC50 of more than 100, as can be seen from [Fig. 2].

This assay allowed to demonstrate that the hydrogen from the amide in the centre of the molecule is crucial for the enzymatic affinity and is probably involved in an H-bond with the protein. Indeed, VRO001_hydrol proved unable to inhibit catB. On the other hand, the hydrogen on the amide next to the isoamylamine moiety is not involved in the binding since the activity from VRO006 is quite similar to the one of E64c.

All modifications made on R₁ were well tolerated. The methoxy compound VRO047_hydrol showed almost the same activity as E64c. The same applies for VRO052_hydrol and VRO059_hydrol as well as for VRO073_hydrol. Even with bulky aromatic moieties, like in VRO052_hydrol and VRO073_hydrol, the activity is only slightly reduced. However, the tolerance of the S3 pocket of catB has limits. Indeed, the bulky and highly electronegative -CF₃ moiety present in VRO035_hydrol was not accepted and the inhibitory activity was lost for this compound.

Concerning R₃, which binds to the S2 pocket of catB, the tolerance was much lower. Even small changes, like the introduction of an ether in VRO082_hydrol did not allow to maintain the inhibitory activity. The bulkiness from the pyridinyl moiety added in VRO119_hydrol was also not tolerated. However, one candidate created the surprise, especially considering the results obtained with other moieties in position R₃. VRO109_hydrol includes a trifluoro moiety instead of the leucine present in E64c. Adding such a trifluoro moiety in position R₁ resulted in loss of the inhibitory activity, whereas several other moieties, and even bulky ones managed to keep such activity. Now, surprisingly it was demonstrated that VRO109_hydrol exhibits an inhibitory activity that is increased compared to E64c. From all the candidates tested herein VRO109_hydrol is the only one to exhibit a small but significant increase in inhibitory activity compared to the prior art E64c. The trifluoro moiety is particularly advantageous, as it is not susceptible of generating a hydrogen-bond and significantly increases the lipophilicity of the compound compared to E64c.

The assay performed with VRO244_hydrol provides evidence that the trifluoro moiety in position R3 is compatible with a bulky moiety in position R₁ (in this case 2-fluorophenylmethyl).

Individually, the replacement of leucine by a trifluoro moiety and the methylation of the amide close to isoamylamine moiety showed promising results with respect to both activity and drug-likeness, as explained above. However, their combination in VRO243_hydrol implied a drastic loss of activity. This certainly results from the 3D conformation of the enzyme's catalytic site and how the trifluoro moiety is positioned into it. This particular 3D layout is probably incompatible with the one obtained when the nitrogen is methylated.

### Example 10: In-vitro whole cell enzymatic activity assays

To confirm the results obtained on the isolated catB (see Example 9), the inhibitory activity of compounds of the invention on catB was assessed in a whole cell assay. In this case the free acid (hydrolyzed) form of the compounds of the invention was used.

Various concentration (50, 10, 2, 0.4, 0.08, 0.016, 0.0032 et 0 mM) of test substances were placed in 96 well plate and 50'000 HEK293T cells were added to each well. The plates were then incubated at 37°C for one hour. The plates were centrifuged for 7 minutes at 2000 rpm, and the supernatant was removed. 100ml of freshly prepared Z-FR-AMC substrate containing 1 M sodium acetate pH 6.0, 2.5 mM EDTA, 2.5 mM DTT, 0.1% Triton X-100 and Z-FR-AMC 20 mM. Following a 1-hour incubation, fluorescence was determined at 445 nm using 365 nm as the excitation wavelength.

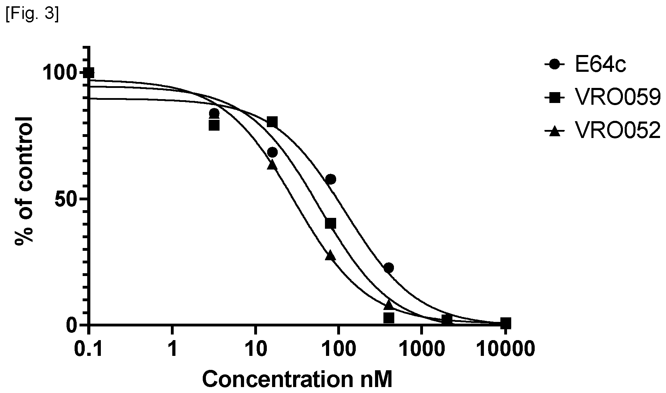

As shown in [Fig. 3], the inhibitory activity of VRO052_hydrol and VRO059_hydrol was confirmed in the whole cell assay. Such activity was even closer to that of E64c than in the assay on isolated enzyme. Even though the activity of VRO052_hydrol and VRO059_hydrol was slightly lower than that of E64c in the assay with the isolate enzyme these compounds exhibit an inhibitory activity similar to that of E64c in the whole cell assay. This improvement of the relative inhibitory activity of VRO052_hydrol and VRO059_hydrol is believed to be due to the fact that the compounds of the invention are more lipophilic and thus enter the cell more easily by crossing the cell membrane than the prior art compound. Thus, this trial confirms that the improved lipophilicity compensates for the slight reduction of inhibitory activity on the isolated enzyme. Based on these findings, it is expected that VRO052_hydrol and VRO059_hydrol exhibit an inhibitory activity similar to E64d in vivo.

## Claims

1. A compound of Formula I or of Formula II 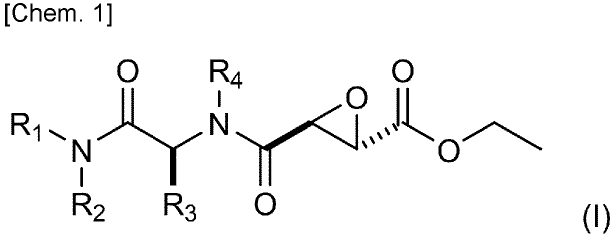 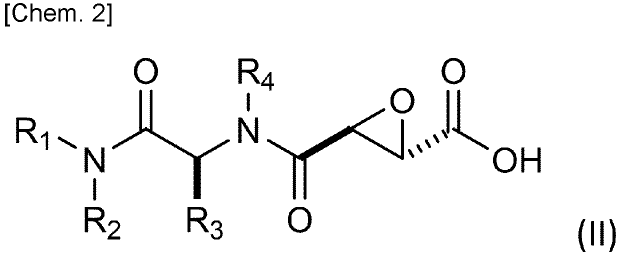 wherein
R₁ is selected from 3-methylbutyl, 2-methoxyethyl, 2-fluorophenylmethyl, 3-(piperidin-1-yl)propyl and 2-(pyridin-2-yl)ethyl.
R₂ is selected from hydrogen and methyl;
R₃ is selected from 2-methylpropyl and 2,2,2-trifluoroethyl; and
R₄ is hydrogen;
said compound being **characterized in that**, when R₁ is 3-methylbutyl,
i. R₃ is not 2-methylpropyl; or
ii. R₃ is 2-methylpropyl and R₂ is methyl,
said compound being further **characterized in that**, when R₃ is 2,2,2-trifluoroethyl, R₂ is hydrogen.

2. A compound according to claim 1, wherein R₁ is selected from 3-methylbutyl, 3-(piperidin-1-yl)propyl and 2-(pyridin-2-yl)ethyl.

3. A compound according to claim 1, wherein R₁ is 3-(piperidin-1-yl)propyl or 2-(pyridin-2-yl)ethyl and R₃ is 2-methylpropyl or wherein R₄ is 3-methylbutyl and R₃ is 2,2,2-trifluoroethyl.

4. A compound according to claim 1, wherein the compound of Formula I
is selected from the group consisting of ethyl (2S,3S)-3-(((S)-1-(isopentyl(methyl)amino)-4-methyl-1-oxopentan-2-yl)carbamoyl)oxirane-2-carboxylate, ethyl (2S,3S)-3-(((S)-4-methyl-1-oxo-1-((2-(pyridin-2-yl)ethyl)amino)pentan-2-yl)carbamoyl)oxirane-2-carboxylate, ethyl (2S,3S)-3-(((S)-4-methyl-1-oxo-1-((3-(piperidin-1-yl)propyl)amino)pentan-2-yl)carbamoyl)oxirane-2-carboxylate, ethyl (2S,3S)-3-(((S)-1-((2-2-methoxyethyl)amino)-4-methyl-1-oxopentan-2-yl)carbamoyl)oxirane-2-carboxylate, ethyl (2S,3S)-3-(((S)-1-((2-fluorobenzyl)amino)-4-methyl-1-oxopentan-2-yl)carbamoyl)oxirane-2-carboxylate, ethyl (2S,3S)-3-(((S)-4,4,4-trifluoro-1-(isopentylamino)-1-oxobutan-2-yl)carbamoyl)oxirane-2-carboxylate and ethyl (2S,3S)-3-(((S)-4,4,4-trifluoro-1-((2-fluorobenzyl)amino)-1-oxobutan-2-yl)carbamoyl)oxirane-2-carboxylate, preferably from the group consisting of ethyl (2S,3S)-3-(((S)-4-methyl-1-oxo-1-((2-(pyridin-2-yl)ethyl)amino)pentan-2-yl)carbamoyl)oxirane-2-carboxylate, ethyl (2S,3S)-3-(((S)-4-methyl-1-oxo-1-((3-(piperidin-1-yl)propyl)amino)pentan-2-yl)carbamoyl)oxirane-2-carboxylate and ethyl (2S,3S)-3-(((S)-4,4,4-trifluoro-1-(isopentylamino)-1-oxobutan-2-yl)carbamoyl)oxirane-2-carboxylate,
or wherein the compound of Formula II is selected from the group consisting of (2S,3S)-3-(((S)-1-(isopentyl(methyl)amino)-4-methyl-1-oxopentan-2-yl)carbamoyl)oxirane-2-carboxylic acid, (2S,3S)-3-(((S)-4-methyl-1-oxo-1-((2-(pyridin-2-yl)ethyl)amino)pentan-2-yl)carbamoyl)oxirane-2-carboxylic acid, (2S,3S)-3-(((S)-4-methyl-1-oxo-1-((3-(piperidin-1-yl)propyl)amino)pentan-2-yl)carbamoyl)oxirane-2-carboxylic acid, (2S,3S)-3-(((S)-1-((2-2-methoxyethyl)amino)-4-methyl-1-oxopentan-2-yl)carbamoyl)oxirane-2-carboxylic acid, (2S,3S)-3-(((S)-1-((2-fluorobenzyl)amino)-4-methyl-1-oxopentan-2-yl)carbamoyl)oxirane-2-carboxylic acid, (2S,3S)-3-(((S)-4,4,4-trifluoro-1-(isopentylamino)-1-oxobutan-2-yl)carbamoyl)oxirane-2-carboxylic acid and (2S,3S)-3-(((S)-4,4,4-trifluoro-1-((2-fluorobenzyl)amino)-1-oxobutan-2-yl)carbamoyl)oxirane-2-carboxylic acid, preferably from the group consisting of (2S,3S)-3-(((S)-4-methyl-1-oxo-1-((2-(pyridin-2-yl)ethyl)amino)pentan-2-yl)carbamoyl)oxirane-2-carboxylic acid, (2S,3S)-3-(((S)-4-methyl-1-oxo-1-((3-(piperidin-1-yl)propyl)amino)pentan-2-yl)carbamoyl)oxirane-2-carboxylic acid and (2S,3S)-3-(((S)-4,4,4-trifluoro-1-(isopentylamino)-1-oxobutan-2-yl)carbamoyl)oxirane-2-carboxylic acid.

5. A compound according to any one of claims 1 to 4, for use in therapy.

6. A compound of Formula I or of Formula II 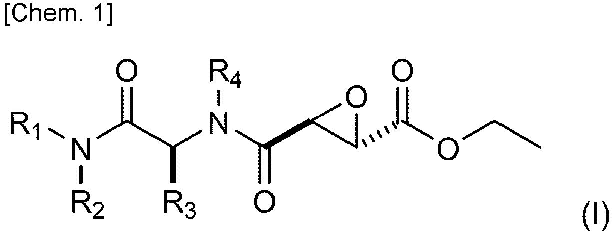 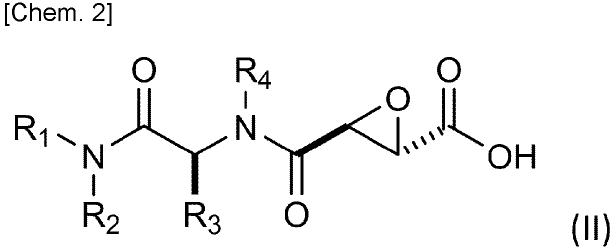 wherein
R₁ is selected from 3-methylbutyl, 2-methoxyethyl, 3-methoxypropyl, 2-fluorophenylmethyl, 3-(piperidin-1-yl)propyl and 2-(pyridin-2-yl)ethyl.
R₂ is selected from hydrogen and methyl;
R₃ is selected from 2-methylpropyl and 2,2,2-trifluoroethyl; and
R₄ is hydrogen;
said compound being **characterized in that**, when R₁ is 3-methylbutyl,
i. R₃ is not 2-methylpropyl; or
ii. R₃ is 2-methylpropyl and R₂ is methyl,
said compound being further **characterized in that**, when R₃ is 2,2,2-trifluoroethyl, R₂ is hydrogen,
for use in a method for treating a disease associated with impaired β-galactosidase activity.

7. A compound for use according to claim 6, wherein said compound of Formula I or of Formula II is as defined in any one of claims 2 to 4 or wherein said compound of Formula I is ethyl (2S,3S)-3-(((S)-1-((3-3-methoxypropyl)amino)-4-methyl-1-oxopentan-2-yl)carbamoyl)oxirane-2-carboxylate and said compound of Formula II is (2S,3S)-3-(((S)-1-((3-3-methoxypropyl)amino)-4-methyl-1-oxopentan-2-yl)carbamoyl)oxirane-2-carboxylic acid.

8. A compound for use according to claim 6 or 7, wherein the disease associated with impaired β-galactosidase activity is selected from GM-1 gangliosidosis, Morquio syndrome type B, Chediak-Higashi Syndrome, Galactosialidosis, Metachromatic leukodystrophy, Gaucher Disease, Alzheimer disease and traumatic brain injury.

9. A compound for use according to claim 8, wherein the disease associated with impaired β-galactosidase activity is selected from GM-1 gangliosidosis, Morquio syndrome type B, Chediak-Higashi Syndrome, Galactosialidosis, Metachromatic leukodystrophy and Gaucher Disease.

10. A compound for use according to any one of claims 6 to 9, wherein the compound is for use in a method for treating a disease associated with impaired β-galactosidase activity, by inhibition of the activity of cathepsin B.

11. A compound for use according to any one of claims 5 to 10, wherein the compound is of Formula I and wherein said compound is administered enterally to a patient in need thereof.

12. A compound for use according to any one of claims 5 to 10, wherein the compound is of Formula II and wherein said compound is administered to a patient in need thereof by an administration route that is not enteral administration.

13. A compound for use according to any one of claims 5 to 12, wherein the compound of Formula I or of Formula II is administered to a patient in need thereof in combination with at least one additional compound selected from a pharmacological chaperone of the β-galactosidase, a pharmacological chaperone of the glucocerebrosidase, a calcium channel blocker, a glucosylceramide synthase inhibitor and mixtures thereof.

14. A compound for use according to claim 13, wherein said at least one additional compound is selected from the group consisting of N-substituted 5-amino-1-hydroxymethyl-cyclopentanetriols, N-octyl-4-epi-beta-valienamine (NOEV), ambroxol, *cis*-(+)-[2-(2-dimethylaminoethyl)-5-(4-methoxyphenyl)-3-oxo-6-thia-2-azabicyclo[5.4.0]undeca-7,9,11-trien-4-yl]ethanoate (Diltiazem), [(3*S*)-1-azabicyclo[2.2.2]octan-3-yl] *N*-[2-[2-(4-fluorophenyl)-1,3-thiazol-4-yl]propan-2-yl]carbamate (Venglustat), an iminosugar and mixtures thereof, wherein such iminosugar is preferably selected from the group consisting of N-butyl-deoxygalactonojirimycin (Migalastat), 4-epi-isofagomine, 5a-C-pentyl 4-epi-isofagomine, 5a-C-methyl 4-epi-isofagomine, 1,5-dideoxy-1,5-imino-(L)-ribitol (DIR), 5-C-alkyl-imino-L-ribitol, N-(dansylamino)hexylaminocarbonylpentyl-1,5-dideoxy-1,5-imino-D-galactitol, 5N,6S-(N'-butyliminomethylidene)-6-thio-1-deoxygalactonojirimycin (6S-NBI-DGJ), N-nonyl-deoxygalactonojirimycin, N-butyldeoxynojirimycin (Miglustat), isofagomine (Afegostat), AZ-3102 and mixtures thereof.

15. A process for producing a compound according to any one of claims 1 to 4, comprising the steps of:
a) deprotonating the compound of Formula III with a base, such as N,N-Diisopropylethylamine (DIPEA); 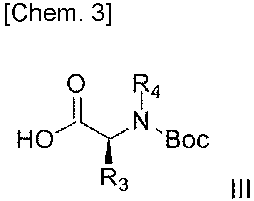
b) reacting the deprotonated compound obtained in step a) with 1-[Bis(dimethylamino)methylene]-1H-1,2,3- triazolo[4,5-b]pyridinium 3-oxid hexafluoro-phosphate (HATU) in basic media;
c) reacting the compounds obtained in step b) with tetramethylurea (TMU) in basic media;
d) reacting the compound obtained in step c) with an amine of Formula IV in basic media, such as to form the dipeptide of Formula V; 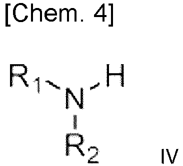 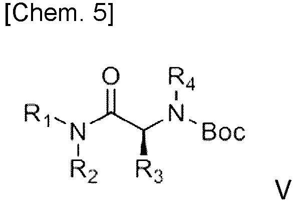
e) removing the Boc group from the compound of Formula V by reacting such compound of Formula V with trifluoroacetic acid in acidic media to obtain the compound of Formula VI; 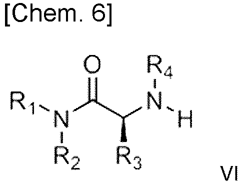
f) deprotonating the compound of Formula VI with a base, such as DIPEA;
g) reacting the deprotonated compound obtained in step f) with HATU in basic media;
h) reacting the compounds obtained in step g) with TMU in basic media;
i) reacting the compounds obtained in step h) with a compound of Formula VII, such as to obtain a compound of Formula I; and 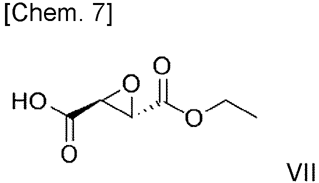
j. optionally hydrolysing the ester moiety of the compound of Formula I, such as to obtain a compound of Formula II.

## Patentansprüche

1. Verbindung der Formel I oder der Formel II 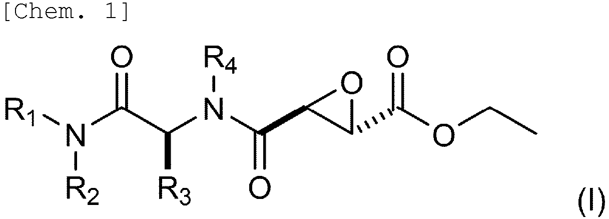 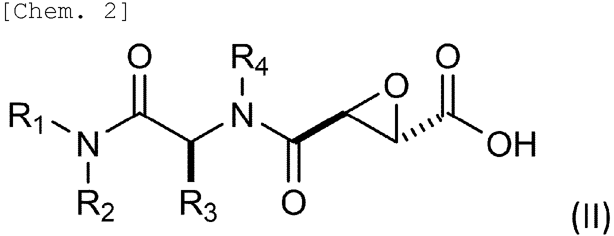 wobei
R₁ aus 3-Methylbutyl, 2-Methoxyethyl, 2-Fluorophenylmethyl, 3-(Piperidin-1-yl)propyl und 2-(Pyridin-2-yl)ethyl ausgewählt ist.
R₂ aus Wasserstoff und Methyl ausgewählt ist;
R₃ aus 2-Methylpropyl und 2,2,2-Trifluoroethyl ausgewählt ist; und
R₄ Wasserstoff ist;
wobei die Verbindung **dadurch gekennzeichnet ist, dass**, wenn R₁ 3-Methylbutyl ist,
i. R₃ nicht 2-Methylpropyl ist; oder
ii. R₃ 2-Methylpropyl ist und R₂ Methyl ist,
wobei die Verbindung ferner **dadurch gekennzeichnet ist, dass**, wenn R₃ 2,2,2-Trifluoroethyl ist, R₂ Wasserstoff ist.

2. Verbindung nach Anspruch 1, wobei R₁ aus 3-Methylbutyl, 3-(Piperidin-1-yl)propyl und 2-(Pyridin-2-yl)ethyl ausgewählt ist.

3. Verbindung nach Anspruch 1, wobei R₁ 3-(Piperidin-1-yl)propyl oder 2-(Pyridin-2-yl)ethyl ist und R₃ 2-Methylpropyl ist oder wobei R₁ 3-Methylbutyl ist und R₃ 2,2,2-Trifluoroethyl ist.

4. Verbindung nach Anspruch 1, wobei die Verbindung der Formel I aus der Gruppe ausgewählt ist, die aus Ethyl-(2S,3S)-3-(((S)-1-(isopentyl(methyl)amino)-4-methyl-1-oxopentan-2-yl)carbamoyl)oxiran-2-carboxylat, Ethyl-(2S,3S)-3-(((S)-4-methyl-1-oxo-1-((2-(pyridin-2-yl)ethyl)amino)pentan-2-yl)carbamoyl)oxiran-2-carboxylat, Ethyl-(2S,3S)-3-(((S)-4-methyl-1-oxo-1-((3-(piperidin-1-yl)propyl)amino)pentan-2-yl)carbamoyl)oxiran-2-carboxylat, Ethyl-(2S,3S)-3-(((S)-1-((2-2-methoxyethyl)amino)-4-methyl-1-oxopentan-2-yl)carbamoyl)oxiran-2-carboxylat, Ethyl-(2S,3S)-3-(((S)-1-((2-fluorobenzyl)amino)-4-methyl-1-oxopentan-2-yl)carbamoyl)oxiran-2-carboxylat, Ethyl-(2S,3S)-3-(((S)-4,4,4-trifluoro-1-(isopentylamino)-1-oxobutan-2-yl)carbamoyl)oxiran-2-carboxylat und Ethyl-(2S,3S)-3-(((S)-4,4,4-trifluoro-1-((2-fluorobenzyl)amino)-1-oxobutan-2-yl)carbamoyl)oxiran-2-carboxylat besteht, vorzugsweise aus der Gruppe ausgewählt ist, die aus Ethyl-(2S,3S)-3-(((S)-4-methyl-1-oxo-1-((2-(pyridin-2-yl)ethyl)amino)pentan-2-yl)carbamoyl)oxiran-2-carboxylat, Ethyl-(2S,3S)-3-(((S)-4-methyl-1-oxo-1-((3-(piperidin-1-yl)propyl)amino)pentan-2-yl)carbamoyl)oxiran-2-carboxylat und Ethyl-(2S,3S)-3-(((S)-4,4,4-trifluoro-1-(isopentylamino)-1-oxobutan-2-yl)carbamoyl)oxiran-2-carboxylat besteht,
oder wobei die Verbindung der Formel II aus der Gruppe ausgewählt ist, die aus (2S,3S)-3-(((S)-1-(isopentyl(methyl)amino)-4-methyl-1-oxopentan-2-yl)carbamoyl)oxiran-2-carbonsäure, (2S,3S)-3-(((S)-4-methyl-1-oxo-1-((2-(pyridin-2-yl)ethyl)amino)pentan-2-yl)carbamoyl)oxiran-2-carbonsäure, (2S,3S)-3-(((S)-4-methyl-1-oxo-1-((3-(piperidin-1-yl)propyl)amino)pentan-2-yl)carbamoyl)oxiran-2-carbonsäure, (2S,3S)-3-(((S)-1-((2-2-methoxyethyl)amino)-4-methyl-1-oxopentan-2-yl)carbamoyl)oxiran-2-carbonsäure, (2S,3S)-3-(((S)-1-((2-fluorobenzyl)amino)-4-methyl-1-oxopentan-2-yl)carbamoyl)oxiran-2-carbonsäure, (2S,3S)-3-(((S)-4,4,4-trifluoro-1-(isopentylamino)-1-oxobutan-2-yl)carbamoyl)oxiran-2-carbonsäure und (2S,3S)-3-(((S)-4,4,4-trifluoro-1-((2-fluorobenzyl)amino)-1-oxobutan-2-yl)carbamoyl)oxiran-2-carbonsäure besteht, vorzugsweise aus der Gruppe, die aus (2S,3S)-3-(((S)-4-methyl-1-oxo-1-((2-(pyridin-2-yl)ethyl)amino)pentan-2-yl)carbamoyl)oxiran-2-carbonsäure, (2S,3S)-3-(((S)-4-methyl-1-oxo-1-((3-(piperidin-1-yl)propyl)amino)pentan-2-yl)carbamoyl)oxiran-2-carbonsäure and (2S,3S)-3-(((S)-4,4,4-trifluoro-1-(isopentylamino)-1-oxobutan-2-yl)carbamoyl)oxiran-2-carbonsäure besteht.

5. Verbindung nach einem der Ansprüche 1 bis 4, zur Verwendung in der Therapie.

6. Verbindung der Formel I oder der Formel II 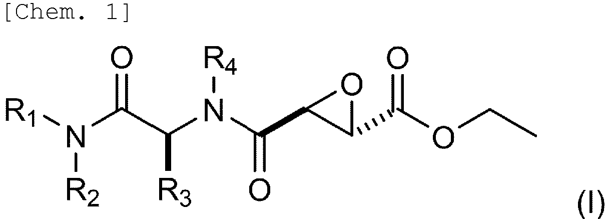 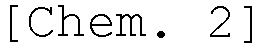 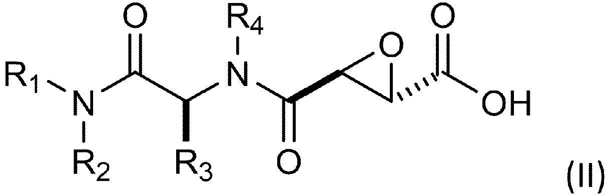 wobei
R₁ aus 3-Methylbutyl, 2-Methoxyethyl, 3-Methoxypropyl, 2-Fluorophenylmethyl, 3-(Piperidin-1-yl)propyl und 2-(Pyridin-2-yl)ethyl ausgewählt ist,
R₂ aus Wasserstoff und Methyl ausgewählt ist;
R₃ aus 2-Methylpropyl und 2,2,2-Trifluoroethyl ausgewählt ist; und
R₄ Wasserstoff ist;
wobei die Verbindung **dadurch gekennzeichnet ist, dass**, wenn R₁ 3-Methylbutyl ist,
i. R₃ nicht 2-Methylpropyl ist; oder
ii. R₃ 2-Methylpropyl ist und R₂ Methyl ist,
wobei die Verbindung ferner **dadurch gekennzeichnet ist, dass**, wenn R₃ 2,2,2-Trifluoroethyl ist, R₂ Wasserstoff ist,
zur Verwendung in einem Verfahren zur Behandlung einer Erkrankung, die mit eingeschränkter β-Galactosidase-Aktivität assoziiert ist.

7. Verbindung zur Verwendung nach Anspruch 6, wobei die Verbindung der Formel I oder der Formel II wie in einem der Ansprüche 2 bis 4 definiert ist, oder wobei die Verbindung der Formel I Ethyl-(2S,3S)-3-(((S)-1-((3-3-methoxypropyl)amino)-4-methyl-1-oxopentan-2-yl)carbamoyl)oxiran-2-carboxylat ist und die Verbindung der Formel II (2S,3S)-3-(((S)-1-((3-3-methoxypropyl)amino)-4-methyl-1-oxopentan-2-yl)carbamoyl)oxiran-2-carbonsäure ist.

8. Verbindung zur Verwendung nach Anspruch 6 oder 7, wobei die Erkrankung, die mit eingeschränkter β-Galactosidase-Aktivität assoziiert ist, aus GM-1-Gangliosidose, Morquio-Syndrom Typ B, Chediak-Higashi-Syndrom, Galaktosialidose, metachromatischer Leukodystrophie, Gaucher-Syndrom, Alzheimer-Krankheit und traumatischen Hirnverletzungen ausgewählt ist.

9. Verbindung zur Verwendung nach Anspruch 8, wobei die Erkrankung, die mit eingeschränkter β-Galactosidase-Aktivität assoziiert ist, aus GM-1-Gangliosidose, Morquio-Syndrom Typ B, Chediak-Higashi-Syndrom, Galaktosialidose, metachromatischer Leukodystrophie und Gaucher-Syndrom ausgewählt ist.

10. Verbindung zur Verwendung nach einem der Ansprüche 6 bis 9, wobei die Verbindung zur Verwendung in einem Verfahren zur Behandlung einer Erkrankung, die mit eingeschränkter β-Galactosidase-Aktivität assoziiert ist, durch Hemmung der Aktivität von Cathepsin B vorgesehen ist.

11. Verbindung zur Verwendung nach einem der Ansprüche 5 bis 10, wobei die Verbindung jene der Formel I ist und wobei die Verbindung enteral einem Patienten, der sie benötigt, verabreicht wird.

12. Verbindung zur Verwendung nach einem der Ansprüche 5 bis 10, wobei die Verbindung jene der Formel II ist und wobei die Verbindung einem Patienten, der sie benötigt, auf einem Verabreichungsweg verabreicht wird, der nicht die enterale Verabreichung ist.

13. Verbindung zur Verwendung nach einem der Ansprüche 5 bis 12, wobei die Verbindung der Formel I oder der Formel II einem Patienten, der sie benötigt, in Kombination mit zumindest einer zusätzlichen Verbindung verabreicht wird, die aus einem pharmakologischen Chaperon der β-Galaktosidase, einem pharmakologischen Chaperon der Glucozerebrosidase, einem Kalziumkanalblocker, einem Inhibitor der Glucosylceramidsynthase und Mischungen davon ausgewählt ist.

14. Verbindung zur Verwendung nach Anspruch 13, wobei die zumindest eine zusätzliche Verbindung aus der Gruppe ausgewählt ist, die aus N-substituierten 5-Amino-1-hydroxymethyl-cyclopentantriolen, N-octyl-4-epi-beta-valienamin (NOEV), Ambroxol, *cis*-(+)-[2-(2-Dimethylaminoethyl)-5-(4-methoxyphenyl)-3-oxo-6-thia-2-azabicyclo[5.4.0]undeca-7,9,11-trien-4-yl]ethanoat (Diltiazem), [(3S)-1-Azabicyclo[2.2.2]octan-3-yl]-N-[2-[2-(4-fluorophenyl)-1,3-thiazol-4-yl]propan-2-yl]carbamat (Venglustat), einem Iminozucker und Mischungen davon besteht, wobei ein solcher Iminozucker vorzugsweise aus der Gruppe ausgewählt ist, die aus n-Butyl-deoxygalactonojirimycin (Migalastat), 4-Epi-isofagomin, 5a-C-Pentyl 4-epi-isofagomin, 5a-C-Methyl 4-epi-isofagomin, 1,5-Dideoxy-1,5-imino-(L)-ribitol (DIR), 5-C-Alkyl-imino-L-ribitol, N-(Dansylamino)hexylaminocarbonylpentyl-1,5-dideoxy-1,5-imino-D-galactitol, 5N,6S-(N'-Butyliminomethyliden)-6-thio-1-deoxygalactonojirimycin (6S-NBI-DGJ), N-Nonyl-deoxygalactonojirimycin, N-Butyldeoxynojirimycin (Miglustat), Isofagomin (Afegostat), AZ-3102 und Mischungen davon besteht.

15. Verfahren zur Herstellung einer Verbindung nach einem der Ansprüche 1 bis 4, umfassend die folgenden Schritte:
a) Deprotonieren der Verbindung der Formel III mit einer Base, wie etwa N,N-Diisopropylethylamin (DIPEA); 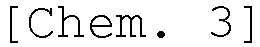 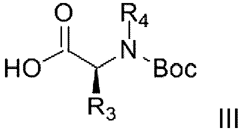
b) Umsetzen der deprotonierten Verbindung, die in Schritt a) erhalten wird, mit 1-[Bis(dimethylamino)methylen]-1H-1,2,3- triazolo[4,5-b]pyridinium-3-oxid-hexafluorophosphat (HATU) in einem basischen Medium;
c) Umsetzen der Verbindungen, die in Schritt b) erhalten werden, mit Tetramethylharnstoff (TMU) in einem basischen Medium;
d) Umsetzen der Verbindung, die in Schritt c) erhalten wird, mit einem Amin der Formel IV in einem basischen Medium, um das Dipeptid der Formel V zu bilden; 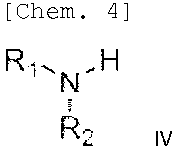 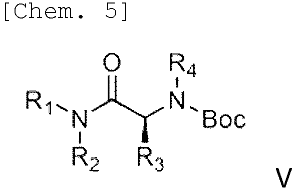
e) Entfernen, der Boc-Gruppe von der Verbindung der Formel V durch Umsetzen der Verbindung der Formel V mit Trifluoressigsäure in einem sauren Medium, um die Verbindung der Formel VI zu erhalten; 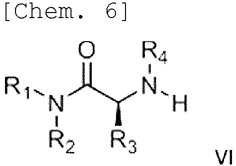
f) Deprotonieren der Verbindung der Formel VI mit einer Base, wie etwa DIPEA;
g) Umsetzen der deprotonierten Verbindung, die in Schritt f) erhalten wird, mit HATU in einem baischen Medium;
h) Umsetzen der Verbindungen, die in Schritt g) erhalten werden, mit TMU in einem basischen Medium;
i) Umsetzen der Verbindungen, die in Schritt h) erhalten wurden, mit einer Verbindung der Formel VII, um eine Verbindung der allgemeinen Formel I zu erhalten; und 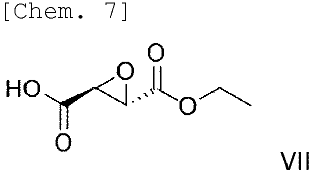
j) gegebenenfalls Hydrolysieren der Ester-Einheit der Verbindung der Formel I, um eine Verbindung der Formel II zu erhalten.

## Revendications

1. Composé de la formule I ou de la formule II 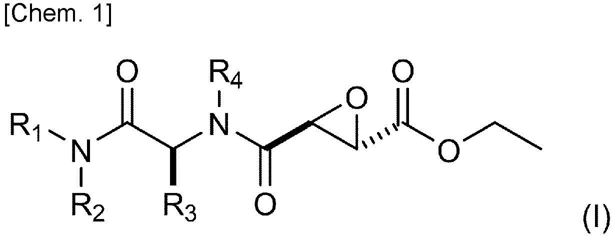 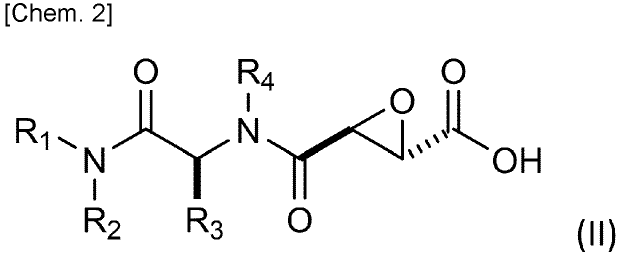 dans lequel
R₁ est choisi parmi le 3-méthylbutyle, le 2-méthoxyéthyle, le 2-fluorophénylméthyle, le 3-(pipéridin-1-yl)propyle et le 2-(pyridin-2-yl)éthyle.
R₂ est choisi parmi l'hydrogène et le méthyle ;
R₃ est choisi parmi le 2-méthylpropyle et le 2,2,2-trifluoroéthyle ; et
R₄ est de l'hydrogène ;
ledit composé étant **caractérisé en ce que**, lorsque R₁ est du 3-méthylbutyle,
i. R₃ n'est pas du 2-méthylpropyle ; ou
ii. R₃ est le 2-méthylpropyle et R₂ est le méthyle,
ledit composé étant en outre **caractérisé en ce que**, lorsque R₃ est du 2,2,2-trifluoroéthyle, R₂ est de l'hydrogène.

2. Composé selon la revendication 1, dans lequel R₁ est choisi parmi le 3-méthylbutyle, le 3-(pipéridin-1-yl)propyle et le 2-(pyridin-2-yl)éthyle.

3. Composé selon la revendication 1, dans lequel R₁ est du 3-(pipéridin-1-yl)propyle ou du 2-(pyridin-2-yl)éthyle et R₃ est du 2-méthylpropyle ou dans lequel R₁ est du 3-méthylbutyle et R₃ est du 2,2,2-trifluoroéthyle.

4. Composé selon la revendication 1, dans lequel le composé de formule I est choisi dans le groupe constitué du (2S,3S)-3-(((S)-1-(isopentyl(méthyl)amino)-4-méthyl-1-oxopentan-2-yl)carbamoyl)oxirane-2-carboxylate d'éthyle, du (2S,3S)-3-(((S)-4-méthyl-1-oxo-1-((2-(pyridin-2-yl)éthyl)amino)pentan-2-yl)carbamoyl)oxirane-2-carboxylate d'éthyle, (2S,3S)-3-(((S)-4-méthyl-1-oxo-1-((3-(pipéridin-1-yl)propyl)amino)pentan-2-yl)carbamoyl)oxirane-2-carboxylate d'éthyle, du (2S,3S)-3-(((S)-1-((2-2-méthoxyéthyl)amino)-4-méthyl-1-oxopentan-2-yl)carbamoyl)oxirane-2-carboxylate d'éthyle, du (2S,3S)-3-(((S)-1-((2-fluorobenzyl)amino)-4-méthyl-1-oxopentan-2-yl)carbamoyl)oxirane-2-carboxylate d'éthyle, (2S,3S)-3-(((S)-4,4,4-trifluoro-1-(isopentylamino)-1-oxobutan-2-yl)carbamoyl)oxirane-2-carboxylate d'éthyle et du (2S,3S)-3-(((S)-4,4,4-trifluoro-1-((2-fluorobenzyl)amino)-1-oxobutan-2-yl)carbamoyl)oxirane-2-carboxylate d'éthyle, de préférence constitué du (2S,3S)-3-(((S)-4-méthyl-1-oxo-1-((2-(pyridin-2-yl)éthyl)amino)pentan-2-yl)carbamoyl)oxirane-2-carboxylate d'éthyle, du (2S,3S)-3-(((S)-4-méthyl-1-oxo-1-((3-(pipéridin-1-yl)propyl)amino)pentan-2-yl)carbamoyl)oxirane-2-carboxylate d'éthyle et du (2S,3S)-3-(((S)-4,4,4-trifluoro-1-(isopentylamino)-1-oxobutan-2-yl)carbamoyl)oxirane-2-carboxylate d'éthyle,
ou dans lequel le composé de Formule II est choisi dans le groupe constitué de l'acide (2S,3S)-3-(((S)-1-(isopentyl(méthyl)amino)-4-méthyl-1-oxopentan-2-yl)carbamoyl)oxirane-2-carboxylique, de l'acide (2S,3S)-3-(((S)-4-méthyl-1-oxo-1-((2-(pyridin-2-yl)éthyl)amino)pentan-2-yl)carbamoyl)oxirane-2-carboxylique, de l'acide (2S,3S)-3-(((S)-4-méthyl-1-oxo-1-((3-(pipéridin-1-yl)propyl)amino)pentan-2-yl)carbamoyl)oxirane-2-carboxylique, de l'acide (2S,3S)-3-(((S)-1-((2-2-méthoxyéthyl)amino)-4-méthyl-1-oxopentan-2-yl)carbamoyl)oxirane-2-carboxylique, de l'acide (2S,3S)-3-(((S)-1-((2-fluorobenzyl)amino)-4-méthyl-1-oxopentan-2-yl)carbamoyl)oxirane-2-carboxylique, de l'acide (2S,3S)-3-(((S)-4,4,4-trifluoro-1-(isopentylamino)-1-oxobutan-2-yl)carbamoyl)oxirane-2-carboxylique et de l'acide (2S,3S)-3-(((S)-4,4,4-trifluoro-1-((2-fluorobenzyl)amino)-1-oxobutan-2-yl)carbamoyl)oxirane-2-carboxylique, de préférence constitué de l'acide (2S,3S)-3-(((S)-4-méthyl-1-oxo-1-((2-(pyridin-2-yl)éthyl)amino)pentan-2-yl)carbamoyl)oxirane-2-carboxylique, (2S,3S)-3-(((S)-4-méthyl-1-oxo-1-((3-(pipéridin-1-yl)propyl)amino)pentan-2-yl)carbamoyl)oxirane-2-carboxylique et de l'acide (2S,3S)-3-(((S)-4,4,4-trifluoro-1-(isopentylamino)-1-oxobutan-2-yl)carbamoyl)oxirane-2-carboxylique.

5. Composé selon l'une quelconque des revendications 1 à 4, destiné à être utilisé en thérapie.

6. Composé de la formule I ou de la formule II 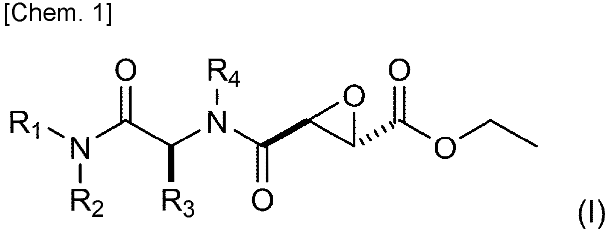 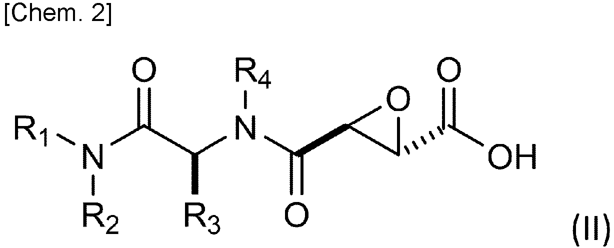
dans lequel R₁ est choisi parmi le 3-méthylbutyle, le 2-méthoxyéthyle, le 3-méthoxypropyle, le 2-fluorophénylméthyle, le 3-(pipéridin-1-yl)propyle et le 2-(pyridin-2-yl)éthyle.
R₂ est choisi parmi l'hydrogène et le méthyle ;
R₃ est choisi parmi le 2-méthylpropyle et le 2,2,2-trifluoroéthyle ; et
R₄ est de l'hydrogène ;
ledit composé étant **caractérisé en ce que**, lorsque R₁ est du 3-méthylbutyle,
i. R₃ n'est pas du 2-méthylpropyle ; ou
ii. R₃ est le 2-méthylpropyle et R₂ est le méthyle,
ledit composé étant en outre **caractérisé en ce que**, lorsque R₃ est du 2,2,2-trifluoroéthyle, R₂ est de l'hydrogène,
pour utilisation dans une méthode de traitement d'une maladie associée à une activité de la β-galactosidase altérée.

7. Composé pour utilisation selon la revendication 6, dans lequel ledit composé de formule I ou de formule II est tel que défini dans l'une quelconque des revendications 2 à 4
ou dans lequel ledit composé de formule I est le (2S,3S)-3-(((S)-1-((3-3-méthoxypropyl)amino)-4-méthyl-1-oxopentan-2-yl)carbamoyl)oxirane-2-carboxylate d'éthyle et ledit composé de formule II et l'acide (2S,3S)-3-(((S)-1-((3-3-méthoxypropyl)amino)-4-méthyl-1-oxopentan-2-yl)carbamoyl)oxirane-2-carboxylique.

8. Composé pour utilisation selon la revendication 6 ou 7, dans lequel la maladie associée à une activité de la β-galactosidase altérée est choisie parmi la gangliosidose GM-1, le syndrome de Morquio de type **B,** le syndrome de Chediak-Higashi, la galactosialidose, la leucodystrophie métachromatique, la maladie de Gaucher, la maladie d'Alzheimer et les lésions cérébrales traumatiques.

9. Composé pour utilisation selon la revendication 8, dans lequel la maladie associée à une activité de la β-galactosidase altérée est choisie parmi la gangliosidose GM-1, le syndrome de Morquio de type B, le syndrome de Chediak-Higashi, la galactosialidose, la leucodystrophie métachromatique et la maladie de Gaucher.

10. Composé pour utilisation selon l'une quelconque des revendications 6 à 9, dans lequel le composé est pour utilisation dans une méthode de traitement d'une maladie associée à une activité de la β-galactosidase altérée, par inhibition de l'activité de la cathepsine B.

11. Composé pour utilisation selon l'une quelconque des revendications 5 à 10, dans lequel le composé est de formule I et dans lequel ledit composé est administré par voie entérale à un patient qui en a besoin.

12. Composé pour utilisation selon l'une quelconque des revendications 5 à 10, dans lequel le composé est de formule II et dans lequel ledit composé est administré à un patient qui en a besoin par une voie d'administration qui n'est pas l'administration entérale.

13. Composé pour utilisation selon l'une quelconque des revendications 5 à 12, dans lequel le composé de formule I ou de formule II est administré à un patient qui en a besoin en association avec au moins un composé supplémentaire choisi parmi un chaperon pharmacologique de la β-galactosidase, un chaperon pharmacologique de la glucocérébrosidase, un inhibiteur calcique, une inhibiteur de la glucosylcéramide synthase et des mélanges de ceux-ci.

14. Composé pour utilisation selon la revendication 13, dans lequel ledit au moins un composé supplémentaire est choisi dans le groupe constitué par les 5-amino-1-hydroxyméthyl-cyclopentanetriols substitués en N, la N-octyl-4-épi-bêta-valienamine (NOEV), l'ambroxol, le *cis*-(+)-[2-(2-diméthylaminoéthyl)-5-(4-méthoxyphényl)-3-oxo-6-thia-2-azabicyclo[5.4.0]undeca-7,9,11-trièn-4-yl]éthanoate (Diltiazem), le [(3S)-1-azabicyclo[2.2.2]octan-3-yl] *N*-[2-[2-(4-fluorophényl)-1,3-thiazol-4-yl]propan-2-yl]carbamate (Venglustat), un iminosucre et leurs mélanges, dans lequel l'iminosucre est choisi de préférence dans le groupe constitué par le N-butyl-désoxygalactonojirimycine (Migalastat), le 4-épi-isofagomine, la 5a-C-pentyl 4-épi-isofagomine, la 5a-C-méthyl 4-épi-isofagomine, le 1,5-didésoxy-1,5-imino-(L)-ribitol (DIR), le 5-C-alkyl-imino-L-ribitol, le N-(dansylamino)hexylaminocarbonylpentyl-1,5-didésoxy-1,5-imino-D-galactitol, le 5N,6S-(N'-butyliminométhylidene)-6-thio-1-désoxygalactonojirimycine (6S-NBI-DGJ), la N-nonyl-désoxygalactonojirimycine, la N-butyldésoxynojirimycine (Miglustat), l'isofagomine (Afegostat), le Z-3102 et leurs mélanges.

15. Procédé de production d'un composé selon l'une quelconque des revendications 1 à 4, comprenant les étapes consistant à :
a) Déprotoner le composé de la Formule III avec une base, telle que la N,N-Diisopropyléthylamine (DIPEA) ; 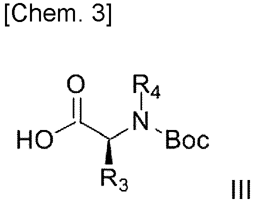
b) faire réagir le composé déprotoné obtenu à l'étape a) avec l'hexafluoro-phospate de 1-[Bis(diméthylamino)méthylène]-1H-1,2,3-triazolo[4,5-b] pyridinium 3 oxyde (HATU) en milieu basique ;
c) faire réagir les composés obtenus à l'étape b) avec de la tétraméthylurée (TMU) en milieu basique ;
d) faire réagir le composé obtenu à l'étape c) avec une amine de formule IV en milieu basique, de sorte qu'ils forment le dipeptide de formule V ; 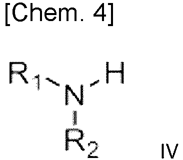 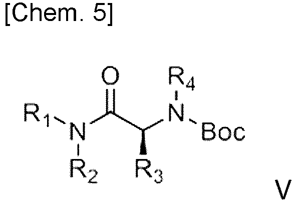
e) éliminer le groupe Boc du composé de formule V en faisant réagir ce composé de formule V avec de l'acide trifluoroacétique en milieu acide pour obtenir le composé de formule VI; 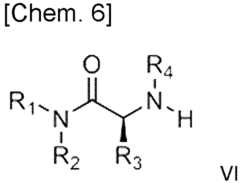
f) déprotoner le composé de formule VI avec une base, telle que la DIPEA ;
g) faire réagir le composé déprotoné obtenu à l'étape f) avec le HATU en milieu basique ;
h) faire réagir les composés obtenus à l'étape g) avec du TMU en milieu basique ;
i) faire réagir les composés obtenus à l'étape h) avec un composé de formule VII, de manière à obtenir un composé de la formule I ; et 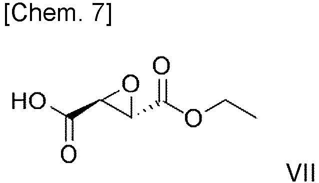
j. de manière optionnelle hydrolyser la fonction ester du composé de formule I, de manière à obtenir un composé de formule II.
